# EUROPEAN PATENT SPECIFICATION

(11) **EP 1 460 986 B1**
(45) Date of publication and mention of the grant of the patent: **14.07.2010**
(21) Application number: 02791139.5
(22) Date of filing: 25.11.2002
(51) Int. Cl.: A61F 13/15

(54) **ABSORBENT ARTICLE WITH IMPROVED FIT**
SAUGFÄHIGER ARTIKEL MIT VERBESSERTEM SITZ
ARTICLE ABSORBANT A ACCESSOIRE AMELIORE

(30) Priority: 06.12.2001 SE 0104086
(43) Date of publication of application: 29.09.2004
(73) Proprietor: SCA Hygiene Products AB, 405 03 Göteborg (SE)
(72) Inventor: WIDLUND, Urban, S-435 43 Pixbo (SE); DREVIK, Solgun, S-435 35 Mölnlycke (SE); ASP, Fredrik, S-439 35 Onsala (SE)
(74) Representative: Olsson, Stefan
(86) International application number: PCT/SE2002/002155
(87) International publication number: WO 2003/059222

(56) References cited:
- EP-A1- 0 298 348
- EP-A2- 0 852 938
- EP-A2- 0 956 844
- EP-A2- 0 965 318
- WO-A1-99/25282
- US-A- 4 886 513
- US-B1- 6 210 385

## Description

### TECHNICAL FIELD

The present invention relates to an absorbent article, such as a sanitary towel, a panty liner, an incontinence pad, a nappy or the like, which article has a longitudinal direction and a transverse direction, a front portion, a rear portion, a crotch portion located between the rear portion and the front portion, an absorbent element and a liquidtight layer, and also a stiffening element which is intended to contribute to the three-dimensional shape of the article during its use.

### BACKGROUND ART

A great many different demands are made of absorbent articles, such as a sanitary towel, an incontinence pad, a nappy or the like, which are not easy to satisfy simultaneously. A fundamental requirement is that the article, for example a sanitary towel, should be capable of catching and absorbing bodily fluid discharged from the wearer. Conventional sanitary towels in sizes intended for heavy flows of menstrual fluid have been of thick and relatively wide design. Sanitary towels of this type are described in, for example, US 3 294 091. Thick and relatively wide sanitary towels of this type theoretically have great absorption capacity but in practice, when the sanitary towel is subjected to compression forces when squeezed together between the thighs of the wearer, much of the take-up capacity and absorption capacity is lost. The sanitary towel is squeezed together into an arbitrary rope-like shape which frequently does not offer a sufficiently large receiving surface for the menstrual fluid discharged, and leakage occurs in the case of heavy flows of menstrual fluid. The sanitary towel can also be pressed together between the thighs of the wearer in such a manner that the side edges of the sanitary towel and the liquidtight layer are folded in over the liquid-permeable surface and in this way reduce the size of the liquid-receiving surface available.

Sanitary towels are intended to be positioned inside a pair of briefs, the design of which may vary. In this connection, sanitary towels can be positioned incorrectly inside the briefs. There is therefore a risk of the sanitary towel being, by mistake, positioned too far forward or too far back or displaced slightly in the lateral direction and therefore of the absorption capacity and receiving surface of the entire sanitary towel not being optimally utilized.

Conventional sanitary towels are generally retained in the briefs of the wearer by means of pressure-sensitive adhesive or friction coatings. The sanitary towel is fitted by the towel being put in place in the briefs, after which the latter are pulled up into position. When fitting the article inside the briefs, however, it is difficult to achieve a positioning which is optimum in relation to the body of the wearer. Use is usually made of the crotch portion of the briefs in order to determine where the sanitary towel should be positioned. As sanitary towels are manufactured in a great many sizes and models, the position and design of the crotch portion provide a particularly uncertain indication of where in the briefs a sanitary towel is to be positioned, and the functioning of the sanitary towel during use is consequently not always as desired.

Another cause of leakage occurring past sanitary towels attached inside the briefs of the wearer is that the sanitary towel moves together with the briefs instead of following the body movements of the wearer. This means that even a sanitary towel which was from the outset positioned correctly in the briefs in relation to the body can be pulled out of this position by the briefs.

In order to attempt to reduce leakage arising as a result of the sanitary towel being pressed together between the legs of the wearer, it has become usual to provide the sanitary towels with special attachment flaps. It is known from, for example, SE 455 688, US 4 285 343, EP 0 130 848, EP 0 134 086 and US 4 608 047 to provide sanitary towels with flexible side flaps or wings projecting from the longitudinal side edges. These are intended to be folded around the edge portions of the briefs of the wearer when the sanitary towel is put on, and to be attached to the outside of the briefs. The side flaps themselves constitute protection against side edge leakage and soiling of the briefs. Moreover, deformation of the absorption body of the sanitary towel is counteracted by virtue of the fact that the sanitary towel is anchored at the leg edges of the briefs and is held extended between these during use.

However, a considerable disadvantage of providing absorbent articles with such attachment flaps is that many wearers find it embarrassing that the attachment flaps are visible on the outside of the briefs. This also means that absorbent articles with such attachment flaps cannot be used when, for example, the wearer is wearing a swimsuit.

Another disadvantage of the attachment flaps is that they are relatively difficult to handle and require many manual operations in order to be fitted correctly around the leg edges of the briefs. Furthermore, especially in the case of attachment flaps which extend quite a long way along the side edges of a sanitary towel, it can be virtually impossible to fold the attachment flaps around the curved leg edges of the briefs without chafing and unattractive creases in the attachment flaps occurring.

A further problem of sanitary towels with attachment flaps is that the functioning of the attachment flaps or wings depends on the design of the briefs. It goes without saying that a sanitary towel with attachment flaps interacts differently with briefs with a wide crotch compared with briefs with a very narrow crotch.

Attachment flaps or wings on sanitary towels protect the leg edges of the briefs from soiling but as emerged above, are far from being an entirely satisfactory solution.

In order to improve leakproofness, EP 0 067 465 has proposed manufacturing a two-part sanitary towel in which the two parts are interconnected only at their end portions. The lower part is fastened in the briefs of the wearer, and the upper part makes contact with the body of the wearer. The idea is that the parts will be able to move slightly in relation to one another, during use. The mobility between the parts is, however, very limited, and the known sanitary towel is still dependent on the movements of the briefs. Furthermore, there is no guarantee that the upper part will be held in contact with the body of the wearer during use.

WO 97/09014 describes another two-part absorbent article in which the two parts are movable in relation to one another. This known article also has limited mobility between the parts and is to a certain extent dependent on the movements of the briefs.

One way of attempting to reduce the risk of edge leakage caused by deformation of the sanitary towel during use is to provide the sanitary towel with a preshaped raised portion, what is known as a hump, which is intended to make contact with the genitals of the wearer during use of the sanitary towel. Discharged bodily fluid can in this way be caught as soon as it leaves the body of the wearer and be absorbed immediately into the article instead of running out over the surface of the latter. A raised portion also makes it easier for the wearer to position the article correctly in relation to the body. French patent publication FR-A-2 653 328 describes a sanitary towel with a hump in the form of a central, longitudinal, cylindrical raised portion.

A common way of creating a raised portion has been quite simply to build it up by arranging a greater quantity of absorption material within the area of the raised portion. As the absorption material used is in most cases what is known as cellulose fluff pulp, however, such a raised portion collapses and loses its shape when it is wetted. In order to produce a raised portion which is sufficiently large in the wet state as well, a raised portion consisting of cellulose fluff pulp must comprise so much absorption material that it is altogether too high, hard and uncomfortable to wear in the dry state.

It is also known to produce an article with a raised portion facing the wearer by positioning a shaping element on top of the absorbent core. The disadvantage is that this interferes with the liquid transport down to the absorbent, liquid-retaining absorption core and that leakage can occur because the shaping element does not have sufficient admission capacity or temporary retention capacity. The use of, for example, a foamed material in the raised portion has been proposed. However, it has proved difficult to produce a foamed structure with sufficiently open pores for good liquid admission into the latter at the same time as the material is to have such great retention capacity that liquid is not pressed out in the event of loading originating from the wearer, for example when the latter sits down.

Another example of a raised portion is described in Swedish patent 507 798. Such a raised portion has a predictable shape, both before and during use, and also keeps its shape irrespective of the movements of the wearer and of the wetting to which it is subjected. The raised portion is anatomically designed, which means that it is relatively narrow in order to project in slightly between the labia of the wearer during use without causing discomfort for the wearer.

Although such a raised portion functions well for its purpose, it has been found that when the raised portion is exposed to large quantities of bodily fluid over a relatively short period of time, there is a risk that some of the liquid will run on the outside of the raised portion and flow out past the side edges of the absorbent article. Such leakage can occur, for example, when the wearer of a sanitary towel has been sitting or lying down for a relatively long period of time and then suddenly rises. This is because, when the wearer is sitting or lying down, a relatively large quantity of menstrual fluid accumulates in the vagina of the wearer. In the event of a sudden change in body position, the entire quantity of accumulated liquid may be discharged at once. A narrow raised portion of the type described in SE 507 798 does not then have a sufficiently large surface to be capable of receiving and absorbing the entire quantity of liquid in one go, for which reason such sudden liquid flows often result in leakage.

EP 0 335 252 and EP 0 335 253 have proposed providing an absorbent article with a deformation element. The deformation element is acted on by the transverse compressive forces between the thighs of a wearer. The purpose of the deformation element is to cause a portion of the article to bulge in the direction of the body of the wearer during use. It is impossible, however, to control or predict entirely the shape the article will adopt for each individual wearer. Moreover, it is not possible to ensure contact between the body of the wearer and the surface of the article, because the degree of bulging is determined entirely by how much the article is compressed in the transverse direction.

US 4 804 380 describes an absorbent article which has a permanent three-dimensional shape. The article has one end portion of flat or concave shape and one end portion provided with a raised portion. The flat or concave end portion is intended to be positioned in front of the mons Veneris of the wearer, and the end portion comprising the raised portion is intended to fit in against the buttocks of the wearer. The three-dimensional design of the article is brought about by folding a fairly stiff absorption body. In order to make the raised portion permanent, the rear side of the article is provided with a glued surface in the end portion which is to have the raised portion. When the raised portion has been formed, it is maintained by means of the glue.

There are absorbent articles on the market which have a permanent, three-dimensional, boat-like shape and in which the outer shell consists of a moulded polymer foam.

A considerable disadvantage of permanent three-dimensional products is that it is difficult to pack a stiff three-dimensional product. Such products require a great deal of space for transport and sale, and it can be embarrassing for a wearer to carry around a sanitary towel or an incontinence pad which it is impossible to fold and therefore cannot be concealed in the hand or in the worst case will not even fit in a handbag.

EP 155 515 describes how an absorbent article, such as a sanitary towel, is provided with a bowl-shaped appearance by virtue of elastic being applied in a pretensioned state at the longitudinal side edges of the article. The use of elastic complicates manufacture, and there is a risk of the intended elastic effect being lost in connection with packing of the article or when the latter is stored in a folded packing state.

It is previously known to design plane absorbent articles which adopt a three-dimensional, essentially bowl-like shape when applied. An example of this is described in US 4 655 759. This discloses an elongate sanitary towel which consists of a layer of absorbent material, a flexible liquidtight outer layer and a liquid-permeable inner layer. The sanitary towel is provided with a pair of channels formed by stamping, the channels being located on both sides of a longitudinal centre axis and extending along a curved path over the absorption material layer. The two paths together form an hourglass-like shape positioned centrally over the towel. Before use, the sanitary towels are essentially plane but, when they are applied to the wearer, they are folded into a bowl-like shape, that is to say with liquid-stopping upright borders outside the channels. One disadvantage of this bowl-like construction is that the borders hold the central portion of the sanitary towel at a distance from the genitals of the wearer, and liquid discharged from the wearer does not flow directly into the absorbent article but can run on the surface, the risk then being obvious that liquid may find an undesirable transport path in the form of a small crease or the like and run straight out of the product in the lateral or longitudinal direction. Stamped channels in an absorption body also have the disadvantage that the liquid spread in the absorption layer is disrupted and that absorption material outside the channels is not utilized, which increases the risk of local over-saturation and attendant leakage from those parts of the absorption layer which are used.

EP 0 298 348 shows absorbent articles provided with reinforcing members. In one of the embodiment shown the reinforcing members are flat before use. In the preferred embodiments the reinforcing member is in a pre-shaped three-dimensional form. The reinforcing members can be cut from flat sheets of polymer and then heat-shaped to the desired curve before placement in the pad. Such three-dimensional articles are difficult to pack.

EP-A-0 852 938 describes articles where the reinforcing or stiffening element is in the form of embossed channels in a conventional absorbent core. Embossed channels are arranged in the cross-direction of the article in both ends thereof. Embossed channels can also be arranged In the middle of the absorbent article. The articles according to EP-A-0 852 938 also have non-stiffened regions and the articles are said to bend in the non-stiffened region and the stiffened element resist transverse bunching.

Previously known sanitary towels and the various problems associated with them have in the main been discussed above. However, what has been said above also applies to incontinence pads. Nappies for children and adults also belong to the same problem area as far as fit in the crotch and take-up of liquid in an absorption body are concerned.

As emerged above, great efforts have been made over many years in order to attempt to solve all the problems associated with absorbent articles, such as sanitary towels. Although great improvements have been made, all the previously known solutions are associated with some disadvantages.

### DISCLOSURE OF INVENTION

By means of the present invention, an improved absorbent article of the type mentioned in the introduction has been produced. The article according to the invention is characterized mainly in that the stiffening area is in a plane state before use of the article and comprises a first stiffening part area in the front portion, in that the first stiffening part area in the front portion has a width which, at least at some point in the longitudinal direction of the article, exceeds the distance between the muscle tendons of the wearer on both sides of the crotch of the wearer in the groin of the latter, which distance is about 25-45 mm, in that the article comprises an elongate absorbent crotch area which is intended to be fitted in the crotch of the wearer over the genitals of the latter and is designed for an optimum absorption function by free selection of absorption material and construction, in that the absorbent crotch area is attached to the first part area and held in place by the latter so as to avoid the absorbent crotch area sliding backwards and out of the intended position over the genitals of the wearer when the wearer moves, in that in the front portion of the article, the side edges of the first stiffening part area diverge in the forward direction from the crotch portion at least part of the way in over the front portion, in that, in the direction from the crotch area, the side edges of the stiffening part element form an acute angle with a line in the longitudinal direction of the article, and in that the first stiffening part area extends into the transition between the front portion and the crotch portion of the article, and in that the first stiffening part area has a width at said transition of in the order of 20-35 mm.

An absorbent article according to the invention has a number of advantages. It is plane before use, and there are therefore no problems associated with packing, storing and transporting said article. The first stiffening part area has a width which at least at some point exceeds the distance between the muscle tendons of the wearer on both sides of the crotch of the wearer in the groin of the latter, which has the advantage compared with conventional absorbent articles, such as sanitary towels, that the article does not move backwards in an uncontrolled manner in the crotch of the wearer when the latter moves and come out of the intended position with reduced functioning as a consequence.

According to one embodiment, the absorbent crotch area is less stiff than the first stiffening part element.

According to a further embodiment, the invention is **characterized in that,** in relation to said stiffening area, said absorbent crotch area is soft and yielding when utilization stresses occur.

According to another embodiment, the invention is **characterized in that** the absorbent crotch element consists of a material which is different from that in the first part area.

It is known that the distance between said muscle tendons is very similar for all people. Fatness of course has an effect on the width between the thighs, but the width between the muscle groups is the same, and it is these which may cause an article to feel as if it chafes. The fat tissue lies on the outside of the muscles but does not contribute to any sensation of discomfort. The distance between said muscle tendons is the same irrespective of whether the wearer is slim, of normal weight or overweight. An absorbent article according to the invention automatically adopts a three-dimensional bowl-like shape in an area in the front portion next to the crotch portion when the article is, with the first stiffening part area, fixed in between said muscle tendons. It has been found that what determines whether a wearer experiences discomfort in the form of pressure or chafing against the insides of the thighs is whether the absorbent article has a width during use which in the critical area considerably exceeds the distance between the muscle tendons in the groin portion and whether the article is stiff in the area which is located directly in front of said muscle tendons. This distance has been found to be roughly 25-45 mm. It has been found that an article with a width which exceeds 40 mm in the critical area during use feels uncomfortable to wear to the majority of wearers. On the other hand, it is rarely experienced as being unpleasant if an absorbent article pushes down or aside fat tissue which may be present in the crotch area of the wearer.

Surprisingly, it has been found that this distance between said muscle tendons does not change throughout the lifetime of a person. Small infants therefore have a corresponding critical distance, which, according to the present invention, can be utilized for producing nappies for infants which fit better during use. The same of course applies for nappies for adults. It should be pointed out that said critical distance between the muscle tendons applies for men also, who have the same distance between said muscle tendons.

An article designed according to the invention is adapted to the anatomy of the wearer. The design of the first stiffening part area with a width which at least at some point exceeds the distance between the muscle tendons of the wearer directly in front of the groins results in an article being anchored firmly in the groins of the wearer during use, and in this way the article is prevented from moving backwards between the legs of the wearer. This is otherwise a common problem in conventional articles because the leg movements of the wearer often shift the article backwards.

According to one embodiment, the article according to the invention is **characterized in that** the absorbent crotch element comprises or consists of an absorbent foamed material. According to another embodiment, the invention is **characterized in that** the absorbent crotch element comprises or consists of cellulose fluff Pulp.

According to one embodiment, the invention is **characterized in that** the absorbent crotch area comprises superabsorbent material which is mixed with a material forming part of the crotch area, such as fluff pulp or absorbent foam; or which is arranged in one or more plies in the absorbent crotch area.

An acute angle has been found to be suitable from the point of view of comfort as, with such an angle, the article can be braked in its backward sliding when the wearer moves without chafing occurring at said muscle tendons in the groin of the wearer.

According to one embodiment, the invention is **characterized in that** said width of the first stiffening part area at the transition between the crotch portion and the front portion is of the order of 25-30 mm.

According to one embodiment, the invention is **characterized in that** a second stiffening part element extends part of the way in over the rear portion of the article, and in that the side edges of the second stiffening part element diverge in the direction from the crotch portion at least part of the way from the crotch portion in over the rear portion of the article.

According to one embodiment, the invention is **characterized in that** the second stiffening part element has a cutout extending from its end edge in the rear portion in the direction towards the crotch portion, as a result of which the article is during use provided with a fold along the longitudinal direction of the article in said cutout, which fold extends into the cleft between the buttocks of the wearer during use of the article.

According to another embodiment, the invention is **characterized in that** the second stiffening part element has an elongate second through-hole which extends in the longitudinal direction of the article and along the centre line of the article, as a result of which the article is during use provided with a fold along the longitudinal direction of the article along said second hole, which fold extends into the cleft between the buttocks of the wearer during use of the article and thus stabilizes the article in position on the wearer.

According to one embodiment, the second part area is made from the same material as the first part area. According to one embodiment, the second part area is made with the same stiffness as the first part element.

The first stiffening part element suitably has a stiffness of in the order of 1-15 N measured according to ASTM D 4032-82. This "Circular Bend Procedure" is described in detail in EP 336 578.

According to one embodiment, the invention is **characterized in that** said stiffening part areas consist of compressed part areas of a continuous material body made in one piece, which forms said absorbent crotch area between said part areas.

According to one embodiment, the invention is in this connection **characterized in that** said stiffening part areas consist of part areas of a body made in one piece from foamed material, which part areas are compressed and bonded in compressed state.

According to another embodiment, the invention is **characterized in that** at least one of said stiffening part areas consists of a separate part element.

According to one embodiment, the invention is **characterized in that** said stiffening part area also serves as an absorbent element and consists of a number of interconnected tissue layers with, arranged between the layers, superabsorbent material in the form of particles or fibres.

According to one embodiment, the invention is **characterized in that** the first stiffening part element consists of a dry-formed fibre mat with a density between 0.15 and 0.75 g/cm³ and a weight per unit area of in the order of 100-400 g/m².

Said dry-formed fibre mat is described in US 5 730 737. The fibre mat produced is very stiff after forming and compression. The fibre mat can be used as it is or be mechanically softened to the desired stiffness.

According to one embodiment, the invention is **characterized in that** the dry-formed fibre mat is provided with the desired reduced stiffness and the desired extensibility by virtue of the degree of compression selected and the compression pattern selected.

A way of very accurately forming thin fibrous webs for use as absorption elements in absorbent articles is described in Swedish patent application 0101393-7. The fibrous webs are formed by air-laying fibres, separate air flows containing fibres being fed to a number n of different mat-forming wheels,-where n is a whole number which is at least 2. Separate web layers are formed on the individual web-forming wheels. The fibrous web is formed by said web layers being combined to form a common fibrous web downstream of the mat-forming wheels, which web has very great manufacturing accuracy by virtue of the manufacturing method.

The manufacturing speed and thus the web speed can be very high, and the desired manufacturing accuracy at the web speed concerned is achieved by selecting a sufficiently high number n of mat-forming wheels. By virtue of this manufacturing method, very thin fibrous webs can be manufactured with very great accuracy.

According to one embodiment, the article according to the invention is **characterized in that** the side edges of the first stiffening part area, which diverge at least part of the way from the crotch portion in over the front portion of the article, are arranged so as to form an angle between a line in the longitudinal direction of the article and each of said side edges of in the order of 35-55°, preferably in the order of 45°. With this geometry in and around the transition between the crotch portion and the front portion, effective anchoring is obtained without the wearer experiencing any discomfort in the form of chafing or the like.

According to one embodiment, the invention is **characterized in that** the absorbent crotch area is attached to the second stiffening part area and held in place by the latter so as to avoid the article sliding forwards when the wearer moves. The expression "attached to" means that the crotch area is directly or indirectly connected to the first stiffening part area and as the case may be the second stiffening part area.

The expression stiffening area means that an area has been reinforced in some way in order that this area is stiffer than the rest of the article. This reinforcement can consist of a separate reinforcing element which also serves as an absorbent element, or a completely separate stiffening element, which has only a stiffening function and can consist of an element, made of paper or plastic for example, which is stiff in relation to the rest of the article and can be constructed from one or more material layers made of the same material or different materials. Alternatively, the stiffening area can be brought about by virtue of the article having been stiffened in this area by extra bonding agent between individual material plies. Alternatively, the article can consist of material which is permanently compressible at least in the area which is to be stiffened, suitable compression taking place during manufacture of the article to bring about the desired stiffness in the area concerned. In the description below, the expressions stiffening area and stiffening element will be used interchangeably, the most suitable expression being selected in order to clarify what is meant at the point concerned in the text. In the patent claims below, however, only the expression stiffening area is used to mean both stiffening area and stiffening element. The absorbent crotch area can consist of a defined area of the article, for example a portion of a larger unit forming part of the article, or of a separate element, that is to say a separate absorbent crotch element.

The absorbent crotch area or crotch element in the article according to the last embodiment is therefore anchored both at the front and at the rear at the transition between the crotch portion and the front portion and, respectively, at the transition between the crotch portion and the rear portion, as a result of which an article which is very stable, well fixed and at the same time comfortable during use is obtained.

According to one embodiment, the invention is **characterized in that** said attachment of the first stiffening part area to the crotch area consists of a connection via a connection element forming part of the article.

According to one embodiment, said connection element consists of the liquidtight layer.

According to one embodiment, the invention is **characterized in that** said connection element consists of an elastically stretchable element, as a result of which the crotch area can move relative to the first part area and, by virtue of the action of the elastic, tends to return to its original position.

According to one embodiment, the invention is **characterized in that** said attachment of the second stiffening part area to the crotch area consists of a connection via a connection element forming part of the article.

According to one embodiment, said connection element then consists of the liquidtight layer.

According to another embodiment, the invention is also **characterized in that** said connection element consists of an elastically stretchable element, as a result of which the crotch element can move relative to the second part element and, by virtue of the action of the elastic, tends to return to its original position.

Further advantageous embodiments of the article according to the invention emerge from the subsequent patent claims.

### DESCRIPTION OF FIGURES

The invention will be described in greater detail below with reference to illustrative embodiments shown in the accompanying drawings, in which:
- Figure 1: shows a plan view of an absorbent article according to a first embodiment;
- Figure 2: shows a section along the line II-II in Figure 1 but in a curved utilization state;
- Figure 3: shows an embodiment, slightly modified in relation to the embodiment according to Figure 1, of an article according to the invention in a plan view;
- Figure 4: shows a section along the line IV-IV in Figure 3;
- Figure 5: shows a plan view of a third embodiment of the article according to the invention;
- Figure 6: shows a plan view of a fourth embodiment of the article according to the invention seen towards that surface of the article which receives bodily fluids;
- Figure 7: shows a plan view of the article according to Figure 6 from the opposite side;
- Figure 8: shows a section along the line VIII-VIII in Figure 6 but in a curved utilization state;
- Figure 9: shows, in perspective and in a utilization state, the article according to the fourth embodiment and also the embodiment shown in Figures 6-8;
- Figure 10: shows a plan view of a fifth embodiment, which is slightly simplified in relation to the embodiment according to Figures 6-9;
- Figure 11: shows a plan view of an absorbent article according to the invention, in the form of a nappy;
- Figure 12: shows a plan view of a sixth embodiment of the article according to the invention seen towards the side which makes contact with the wearer during use of the article;

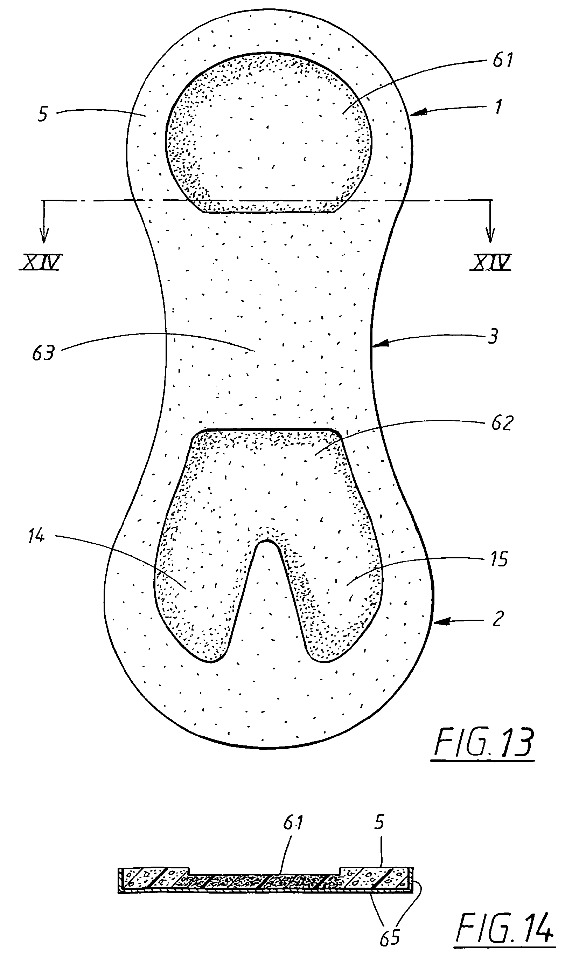

- Figure 13: shows a plan view of a further embodiment of the article according to the invention seen towards that surface of the article which receives bodily fluids, and
- Figure 14: shows a section along the line XIV-XIV in Figure 13.

### MODE(S) FOR CARRYING OUT THE INVENTION

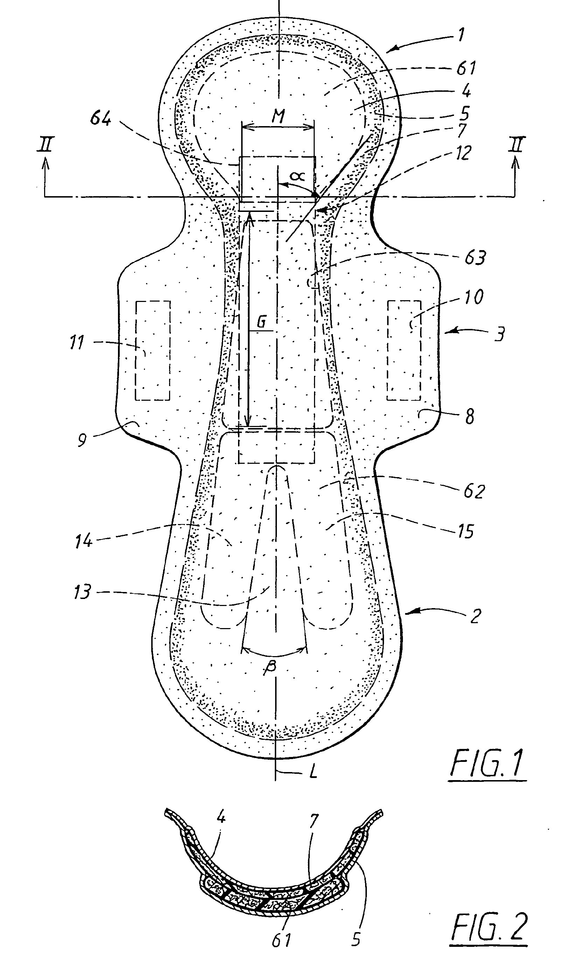

Figures 1 and 2 show an article according to the invention in the form of a sanitary towel or incontinence pad. The article is elongate with a longitudinal direction and a transverse direction. The article has a front portion 1, a rear portion 2 and a crotch portion 3 located between said portions. The article shown in Figures 1 and 2 comprises a liquid-permeable inner layer 4 which is intended to face the wearer during use of the article. The inner layer, which makes contact directly with the skin of the wearer, is suitably made from a soft, textile-like material. Examples of suitable liquid-permeable materials are various types of what are known as non-woven fabrics. Other examples of suitable materials are perforated plastic films. Net and knitted or woven textiles as well as combinations and laminates of said materials can also be used as the inner layer. Examples of inner layers for sanitary towels are laminates of various non-wovens and laminates of non-wovens and perforated plastic films. The liquid-permeable layer can also be integrated with underlying drainage or absorption layers; for example a foam plastic with open pores and with a density gradient in the depth direction can serve as a surface layer and as a drainage layer and/or absorption layer.

The absorbent article also has a liquidtight outer layer 5. This usually consists of a thin plastic layer, made of polyethylene for example. It is also possible to use a liquid-permeable material which has been treated with hydrophobing agent in order to make it liquidtight. In particular if the absorbent article is relatively large, it may be suitable for the outer layer to be breathable and also, if appropriate, vapour-permeable in addition to being liquidtight. Breathable and vapour-permeable but liquid-impermeable materials for use in absorbent articles, such as nappies, incontinence products and sanitary towels, are previously known. Breathable materials can consist of perforated plastic films, which are described in US 5,628,737, or of microporous plastic films, which are described in, for example, EP-A-0238200. Another example of breathable material is what is known as non-woven, which is air and vapour-permeable and may have been treated with agents for improving liquidtightness, as described in EP-A-0196654. In absorbent articles which fit tightly against the wearer, the need for air and vapour-permeability is important.

The absorbent article includes a stiffening element which is designated as a whole by reference number 6 and consists of a first stiffening part element 61 in the front portion and a second stiffening part element 62, separate from the first part element 61, in the rear portion. Arranged between the two part elements 61, 62 is an absorbent crotch element 63 which, in the illustrative embodiment shown here, interacts with said part elements and forms a unit with a keyhole-like shape, which is continuous in the longitudinal direction. The first and second stiffening part elements 61, 62 and the intermediate crotch element are all connected by an elastic element in the form of an elastically stretchable film 64 which bridges a portion of the first part element 61, the crotch element 63 and a portion of the second stiffening part element 62. The elastic element can also consist of longitudinal elastic threads which connect the two stiffening part elements 61, 62 and the intermediate absorbent crotch element 63.

The article also includes a liquid-permeable insulating layer which has a keyhole-like shape but with a greater extent in both the longitudinal direction and the transverse direction than the unit formed by the first and second part elements 61, 62 and the crotch element 63. The outer layer 5 and the inner layer 4 extend with edge portions outside the insulating layer around the latter and are interconnected along these edge portions to form a cover around said continuous unit and the insulating layer 7. In the region of the crotch portion 3, the cover formed by the inner and outer layers extends outwards in the lateral direction to form flexible side flaps 8, 9, what are known as wings, which are intended to be arranged around the crotch portion on the briefs of the wearer in order to protect the edge portions of the briefs from soiling. The wings 8, 9 are suitably provided with adhesive coating, which has been indicated in Figure 1 by reference numbers 10, 11, on the outer layer 5, by means of which the wings can be attached around the crotch portion of the briefs. As can be seen from Figure 2, the insulating layer 7 is located directly inside the inner layer 4 and is principally intended for rapidly admitting discharged bodily fluid into the underlying absorbent crotch element 63 and forming a liquid-insulating layer so as to reduce what is known as back-wetting from the absorbent element 63 to the inner layer 4 making contact directly with the wearer.

The insulating layer can consist of, for example, an airlaid fibrous material of low density bonded together with bonding agent or thermofibre, which is marketed under the designation LDA (low density airlaid). The absorbent crotch element 63 is, seen from the liquid-permeable inner layer 4, arranged under the insulating layer 7. In the illustrative embodiment shown here of the article according to the invention, this element is designed to take up and retain essentially all the bodily fluid discharged. The absorbent crotch element 63 can be made from a material which has smaller capillaries than the insulating layer 7 located above and therefore draws liquid from the insulating layer and prevents back-wetting by liquid from the absorbent crotch element to the insulating element and to the inner layer 4 which remains essentially dry during use of the article. Only when the absorbent crotch element is saturated with liquid can transport take place from the absorbent element to the insulating layer.

The liquid-insulating layer 7 and the absorbent element can of course be made from materials other than those indicated above. The important aspect is that the absorbent element 63 has greater liquid-affinity than the liquid-insulating layer 7 so that liquid is transported from the insulating layer to the absorbent element but not vice versa.

The liquid-insulating layer can consist of, for example, what is known as a multibond non-woven, that is to say a non-woven fabric in which fibres are bonded by both bonding agent and melt bonds. This can also contain fibres or particles made of a slow-acting superabsorbent material and/or an odour-inhibiting superabsorbent material.

In the illustrative embodiment shown, the absorbent crotch element 63 is made less stiff than the stiffening part elements 61, 62 in order to the greatest possible extent to avoid the absorbent crotch element giving rise to chafing when squeezing forces in the lateral direction occur, generated by the thighs of the wearer in the crotch area. The absorbent crotch element 63 is retained in the intended place on the wearer by means of the stiffening part elements 61, 62.

The expression stiffening area means that an area has been reinforced in some way in order that this area is stiffer than the rest of the article. This reinforcement can consist of a separate reinforcing element which can also serve as a supplementary absorbent element, or a completely separate stiffening element which has only a stiffening function and can consist of an element, made of paper or plastic for example, which is stiff in relation to the rest of the article and can be constructed from one or more material layers made of the same material or different materials. Alternatively, the stiffening area can be brought about by virtue of the article having been stiffened in this area by extra bonding agent between individual material plies. Alternatively, the article can consist of material which is permanently compressible at least in the area which is to be stiffened, suitable compression taking place during manufacture of the article to bring about the desired stiffness in the area concerned. The latter illustrative embodiment is described in greater detail below.

As the absorbent crotch element 63 is connected to the stiffening part elements 61 and 62 by means of an elastically stretchable film 64, the crotch element 63 can be moved relative to the first part element 61 and/or the second part element 62 counter to the action of the elastic film 64.

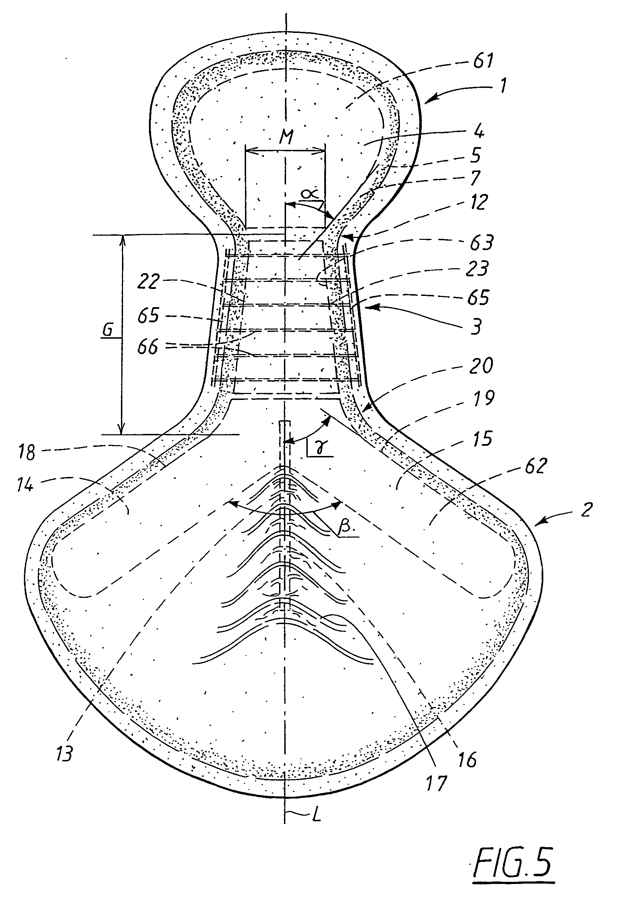

In the article according to the invention, the stiffening part areas (part elements) 61, 62 support the shape of the article during its use and, by virtue of their shape and stiffness, hold the article in the intended place with the first stiffening element 61 in front of the crotch of the wearer and the second stiffening element 62 behind the crotch of the wearer. The absorbent crotch area 63, which can consist of a separate absorbent element 63 as in the illustrative embodiment shown in Figure 5, is designed optimally with regard to the desired absorption capacity as the stiffness properties of the absorbent element are of secondary importance. The essential feature is that the crotch area 63 has great absorption capacity. It is therefore not necessary to compromise on other properties. The absorbent crotch area is suitably designed in such a way that it essentially fills the available space in the crotch of the wearer, so that the volume in the crotch of the wearer is utilized.

At the transition 12 between the crotch portion 3 and the front portion 1, the stiffening part element 61 has a width M which is adapted to the distance between two particular muscle tendons on both sides of the crotch of the wearer directly in front of the groins. These muscle tendons form part of the muscle group which originates on the inside of the pelvic diaphragm and has its attachment along the thigh. This muscle group consists of the adductor brevis, adductor longus, gracilis and adductor magnus muscles. As mentioned above, it is known that this distance between said muscle tendons is very similar for all people. This dimension is of the order of 25-45 mm. Research has shown that 80% of all women have a dimension of roughly 30-32 mm between said muscle tendons. When said width M essentially corresponds to the distance between said muscle tendons of the wearer, the article will be anchored firmly during use with the transition portion between the muscle tendons and be retained in this position. The two side edges of the front portion diverge in the forward direction on the article from said transition area 12. In this way, the article is prevented from moving backwards between the legs of the wearer. This is a common problem in conventional sanitary towels because the leg movements of the wearer often shift the sanitary towel backwards.

In the illustrative embodiment shown, the width M has been marked on that edge of the first stiffening area 61 next to the transition area 12. The width M, as marked in the drawing, can be smaller than the distance between said muscle tendons in the groin of the wearer. The essential feature is that the first stiffening part area has a width somewhere in the longitudinal direction of the article which exceeds said distance between the muscle tendons, so that, during use, the article is braked by the first stiffening area when the wearer moves and the article has a tendency to slide backwards. In the illustrative example shown here, the width varies along the diverging edges of the first stiffening element 61. Diverging edges, as in the illustrative embodiment shown here, are suitable although not necessary for the functioning of the article, but the essential feature is that the width of the first stiffening part area exceeds said distance between the muscle tendons. The first stiffening part element could therefore be, for example, rectangular.

In Figure 1, an angle between a line in the longitudinal direction of the article and each of said side edges has been designated by α. In the case of a large angle α, for example close to 90°, the edges of the front portion may chafe against the groins and legs of the wearer and in this way cause discomfort for the wearer. The smaller the angle α, the greater the risk that the article will slide backwards in between the legs of the wearer. In the case of an angle of less than 30°, this risk is unacceptably high. An angle of 35-45° provides the best balance between secure positioning and comfort. An angle of roughly 45° has been found to be especially favourable.

An absorbent article, such as a sanitary towel, according to the invention is designed with a crotch length adapted to the anatomy of the wearer. In a sanitary towel according to the invention, use has been made of the fact that the great majority of women have a crotch length of in the order of 80-100 mm. The absorbent crotch etement 63 has therefore been designed with a corresponding crotch length G of in the order of 70-120 mm, that is to say the distance from the transition area 12 to the start of the rear portion. Along the crotch, where the body shape of the wearer is essentially plane, the sanitary towel according to the invention can be designed with a certain stiffness in the lateral direction so as not to be deformed in an uncontrolled manner in the lateral direction and form creases. An especially important aspect in an article according to the present invention, however, is that the crotch element 63 is considerably softer and more flexible than the rigid stiffening element 61. As described above, this is fixed between muscle tendons directly in front of the groins of the wearer or alternatively arranged in front of said muscle tendons and in this way secures the crotch element 63 in the intended place. As the crotch element 63 in the embodiment described here also constitutes the major part of the absorption capacity of the sanitary towel, it is essential for it to be possible to utilize available space between the legs of the wearer in the crotch. The width of the sanitary towel in the crotch area is limited at the front by the distance between said muscle tendons directly in front of the groins of the wearer. In the backward direction from said transition area to the end of the crotch portion, the width of the crotch element 63 and thus the absorbent element can increase continuously to of the order of 1.5 times the width in the transition area 12 between the crotch portion and the front portion without any risk of the crotch element being deformed by squeezing together and chafing the wearer in the crotch.

The absorbent crotch element 63 can consist of an absorbent foamed material which is elastically compressible and thus, after squeezing together, tends to return to its original shape. The absorbent crotch element can also be made from a combination of a foamed material and one or more other materials.

The absorbent crotch element 63 can also be made of cellulose fluff pulp, if appropriate in combination with other materials, such as highly absorbent material.

The design described above of the area in and around the transition area 12, that is to say the width of the first stiffening part element or part area, the design of the first part element, the crotch length G selected for the crotch element 63, the size of the angle α and also of the second stiffening element 62 for the article according to the invention, gives the article a good fit and stability in the fitted position on the wearer. This is of particularly great importance for the functioning of the article, not least because the wetting point can vary on account of the body position of the genitals of the wearer in the longitudinal direction of the crotch area. As the available space around the wetting point is very limited in width and length, optimum positioning and anchoring in this position of the stiffening and absorbent first part element 61 is necessary. This is achieved by means of the design described above of the article.

The presence of the elastic element, in the form of an elastic film 64 in the illustrative embodiment shown, means that the crotch element 63 can move relative to the first stiffening part element 61 when tensile or torsional stresses occur and subsequently tends to return to its original position by virtue of the action of the elastic.

The anchoring effect is achieved at said muscle tendons even when the width M on the article is slightly less than the distance between said muscle tendons directly in front of the groins. The two edge portions of the front portion diverge in the forward direction, and the article can slide backwards slightly until the edge portions are anchored firmly between said muscle tendons. For good anchoring, the width M of the first stiffening part element should at some point in the longitudinal direction of the article along said first part element exceed 15-45 mm. A width of the first part element at the transition between the latter and the crotch area of suitably of the order of 15-35 mm and preferably between 25 and 30 mm has been found to function well in an embodiment of the type shown in Figure 1. The latter distance fits most wearers.

As mentioned above, the article in the embodiment shown in Figure 1 also includes a second stiffening part element 62 which is arranged in the rear portion of the article and extends part of the way in over this portion from the crotch portion. The second stiffening part element 62 has a cutout 13 extending from its end edge in the direction towards the crotch portion, as a result of which the article can fold along a longitudinal line L in the cutout and as a result of which the stiffening element forms legs 14 and 15 which are located on both sides of the cutout and are more flexible than the rest of the second part element. The legs 14 and 15 can be made vertically movable in relation to one another by virtue of the width selected for the cutout. This cutout 13 is very important for the adaptation and flexibility of the article in relation to the body. The fold formed in the cutout during use of the article can penetrate the cleft between the buttocks of the wearer and in this way provides very good protection against leakage via the cleft between the buttocks, which type of leakage usually occurs during the use of conventional products when the wearer is lying on her back. The cutout 13 also makes it possible for said legs 14, 15 of the stiffening element to be displaced vertically in relation to one another when various body movements take place, for example when the wearer is walking.

In the illustrative embodiment shown in Figure 1, the cutout 13 is wedge-shaped and located symmetrically in relation to the longitudinal symmetry line L of the article and also forms an angle β of in the order of 20°. This angle can vary within wide limits but of course depends on the design of the rear portion 2. When the rear portion is of considerably wider design, as in the embodiment according to Figure 5, said angle β can vary between of the order of 10° and 120°, preferably between 15° and 40°.

The second stiffening part element 62 can be made of the same material as the first part element 61. The second part element can, irrespective of the material selection in relation to the first part element, be designed with the same stiffness or with a lower level of stiffness. The first part element 61 can be essentially dimensionally stable when utilization stresses occur and yet function without feeling uncomfortable. The second stiffening element 62, however, has to have a certain flexibility in order to function satisfactorily.

In the illustrative embodiment shown, the absorbent crotch element 63 also serves as the main absorption element of the article and suitably also has, in addition to great absorption capacity, great liquid-spreading capacity for rapid spreading of bodily fluid received from the wearer in the narrow crotch area directly in front of the genitals of the wearer over the entire crotch portion. The stiffening part elements 61 and 62 can also be made from liquid-absorbing material. The stiffening part elements can suitably be designed so as to swell in the depth direction during absorption and on the whole retain their geometry in the transverse direction of the article, which results in the stiffening part elements retaining their fit and secure positioning in relation to the body of the wearer throughout use of the article. The stiffening elements 61 and 62 can be designed with very great swelling capacity in the depth direction and attendant great absorption capacity.

According to a suitable embodiment, the stiffening absorbent part elements 61 and 62 consist of a dry-formed fibre mat with a density between 0.15 and 0.75 g/cm³ and a weight per unit area of in the order of 200-400 g/m². A dry-formed fibrous mass in the form of a fibre mat is described in US 5 730 737. The fibre mat produced is very stiff after forming and compression. The fibre mat can be used as it is or be mechanically softened to the desired stiffness.

A way of very accurately forming fibrous webs for use as absorption elements in absorbent articles is described in Swedish patent application 0101393-7. The fibrous webs are formed by air-laying fibres, separate air flows containing fibres being fed to a number n of different mat-forming wheels, where n is a whole number which is at least 2. Separate web layers are formed on the individual web-forming wheels. The fibrous web is formed by said web layers being combined to form a common fibrous web downstream of the mat-forming wheels, which web has very great manufacturing accuracy by virtue of the manufacturing method.

The manufacturing speed and thus the web speed can be very high, and the desired manufacturing accuracy at the web speed concerned is achieved by selecting a sufficiently high number n of mat-forming wheels. By virtue of this manufacturing method, very thin fibrous webs can be manufactured with very great accuracy.

The fibre mat for forming the stiffening absorbent part elements 61 and 62 can consist of a mixture of cellulose fibres and viscose fibres, the presence of the latter giving the fibre mat a greater wet strength than a fibre mat made of only cellulose fibres. The fibre mat for forming the stiffening absorbent part elements can also contain synthetic melt fibres, by means of which the strength of the fibre mat can be increased by heat treatment to melt said synthetic melt fibres.

The absorbent stiffening elements can also be formed from foamed material.

A further example of stiffening absorbent material is a laminate in the form of one or more plies of tissue and superabsorbent material (SAPs). The material or combination of different materials serving as an absorbent element and also, if appropriate, as a stiffening element can contain SAPs in the form of fibres, particles or foam.

The selection of compression pattern also makes it possible to vary the extensibility of the fibre mat. The dry-formed fibre mat can be provided with the desired reduced stiffness and the desired extensibility by virtue of the degree of compression selected and the compression pattern selected.

Furthermore, it is possible to pattern-compress only specific zones for the purpose of providing only these zones with an extensibility and stiffness which are different from the rest of the stiffening absorbent part elements 61 and 62. In the same way, the stiffening part elements 61 and 62 can of course be compressed over their entire extent but with different patterns in different zones. By virtue of the presence of stiffening part elements 61 and 62 which can in a simple manner, by virtue of the pattern compression selected, be provided with the desired stiffness and the desired extension in different zones, and in which the stiffness and extension properties can be selected essentially freely in these zones, the present invention has brought about a new and previously unknown way of controlling and guiding the shaping of an absorbent article intended for taking up bodily fluids.

As mentioned above, a material can be selected for the stiffening part elements 61 and 62, which has great swelling capacity in the depth direction, which has been achieved by great compression of the fibre mat in connection with its production. In the dry state, the fibre mat is hard-compressed and stiff, which affords the shaped and anatomically adapted part elements very good stability in the fitted position on the wearer and very great spreading capacity, as a result of which the total absorption capacity of the stiffening part elements can be optimally utilized and leakage caused by local oversaturation in these areas can to a great extent be eliminated. During absorption of liquid, the stiffening part elements 61 and 62 swell mainly in the depth direction but said part elements do of course swell slightly in other directions as well. When the anatomically adapted stiffening part elements swell, further improved anatomical adaptation is in fact achieved, which contributes to the stability and flexibility of the article in relation to the body shape of the wearer when the stiffness of the stiffening part elements decreases during absorption and attendant swelling.

The first and second stiffening part elements 61 and 62 can be made of non-absorbent material and serve only for securing the article on the wearer and in particular holding the absorbent crotch element 63 in the correct place for optimum functioning.

So as to function in the desired manner, the stiffening element has a stiffness in the dry state of in the order of 1-15 N measured according to ASTM D 4032-82. This "Circular Bend Procedure" is described in detail in EP 336 578.

The stiffening absorbent part element can also consist of a laminate of a number of non-woven fabric layers or tissue layers which are fixed to one another for increased stiffness and which can have highly absorbent material, for example in the form of particles, between individual plies. The individual plies can be fixed to one another by a bonding agent, such as adhesive or melt fibres, using heat or mechanically. The highly absorbent particles can also contribute to bonding. The stiffness is controlled by virtue of the selection of the number of plies and the quantity of bonding agent included and the selection of highly absorbent material and how the adhesive capacity thereof is utilized.

Stiffening absorbent part elements of this type can also be provided with different stiffness and different extensibility in different zones of the extent of the part elements. These properties can in this case as well be controlled by means of compression patterns. This compression can be combined with the supply of heat, which supply can vary in different zones. Furthermore, bonding agent can be applied in different patterns to control the shaping of the stiffening part elements during use. A varying supply of moisture in different areas in connection with compression is another parameter for controlling the shaping of the article during use.

Another example of the construction of a unit serving as both absorption element and stiffening element is a number of layers of LDA, that is to say layers of the same type as in the drainage and insulating layer 7. However, the bonding of the layers of LDA in the stiffening absorption element is much harder within and between individual layers. This bonding is brought about by hard-compression of the LDA layers and suitably by using both melt fibres and latex, what is known as the multibond technique. In this design as well, stiffness and extensibility can be controlled by compression pattern selection and also by variation of the heat supply in different zones.

Further material examples are mixtures of LDA and HDA (high density airlaid) if appropriate in combination with other material layers, such as tissue.

Pattern compression can be used in all the material examples described above, and it is then possible to achieve, for example, hinge effects along compression lines or compression zones.

Pattern formation can take place in connection with compression of the stiffening absorption element. Alternatively, pattern compression can take place in a separate step after smooth compression. Use can be made of, for example, a web of material made in one of the ways described above and smooth-compressed as the starting material for the stiffening absorption element, which is pattern-compressed in the desired manner and depending on the type and size of article to be manufactured. After pattern-compression, individual products are cut out. Pattern-compression and cutting-out of separate stiffening absorption elements can take place in a single step in a combined cutting and pattern-compression unit.

As described above, the stiffening part elements can also serve as an absorption element, that is to say as a complement to the absorbent crotch element 63 of the article.

The invention also includes embodiments in which the stiffening part elements 61 and 62 are non-absorbent. The purpose of such a design is that the part elements should constitute only stiffening shaping elements.

The two stiffening part elements 61, 62 can have different absorption properties. The stiffness can also be different for the first and the second stiffening part element. For example, the front part element can be non-absorbent and have only a stiffening function in order to keep the article in place.

The front stiffening part element 61 can be intended for urine absorption, and the rear stiffening part element 62 can be intended for absorption of motions and menstrual fluid. In such a design, the absorbent crotch element can serve only as a volume reservoir and consist of, for example, a wadding structure which is capable of rapidly taking up large quantities of liquid. Within the scope of the invention, the absorbent crotch element can also consist of a cavity.

In addition to the interpretation of the term stiffening part element as constituting a completely separate element, the term can also embrace the interpretation that all the material plies, bonding agents etc. included in the article in the area of the desired stiffening together form the desired stiffening element. For example, a unit serving as the first stiffening part element, with the dimensions indicated above and with the geometry described above but with stiffness which is in itself inadequate, is included in the invention if the necessary stiffness is obtained by being bonded together with other material plies in the area of this first stiffening part element 61.

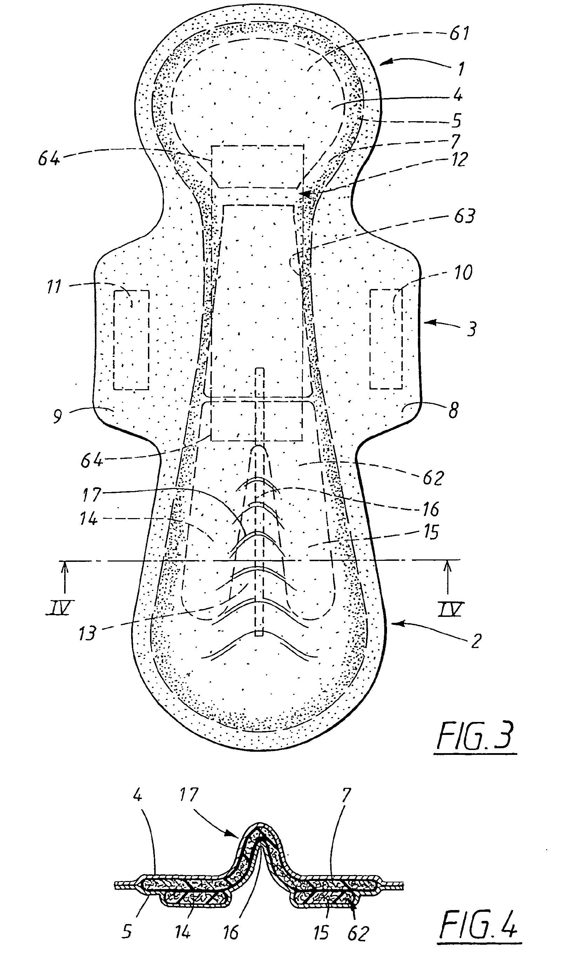

The embodiment shown in Figures 3 and 4 differs from the embodiment shown in Figures 1 and 2 only in that an elastic means 16 is arranged in a pretensioned state in the longitudinal direction of the article and centrally along the rear portion 2 of the article. The same reference numbers have been used in Figures 3 and 4 as in the embodiment according to Figures 1 and 2.

The elastic means 16 is arranged centrally in the cutout and extends in the rear portion slightly beyond the ends of the legs 14 and 15 and in the other direction part of the way in over the crotch portion. The elastic means is arranged on the inside or on the outside of the liquidtight outer layer and is connected to the latter and/or other layers forming part of the article. The extent of the elastic means is not critical but can vary somewhat in relation to the illustrative embodiment shown in Figure 3. One purpose of the elastic means 16 is, during use of the article, to draw adjacent material portions together and curve the article in the upward direction towards the body of the wearer for better contact with the body. Another purpose is also to initiate and form the fold 17 which, during use of the article, is intended to penetrate part of the way into the cleft between the buttocks of the wearer and prevent leakage of bodily fluid backwards along the cleft between the buttocks, which leakage can otherwise occur when the wearer is lying on her back.

In the embodiment shown in Figure 5, the components which correspond to similar parts in the embodiments according to Figures have been provided with the same reference numbers. The article in the embodiment according to Figure 5 is provided with a considerably wider rear portion 2. The article also differs from the embodiments described above in that there are no wings for attachment around the crotch portion of the briefs of the wearer. In the embodiment according to Figure 5, an elastic element connecting the part elements to the absorbent crotch element is not present either. Here, the crotch element is attached to the two stiffening part elements by means of the liquidtight outer layer 5.

The absorbent crotch element 63 and moreover the liquid-insulating layer as well in the area directly in front of the crotch element are enclosed in a covering formed by the liquidtight outer layer 5 and the liquid-permeable inner layer 4. In the embodiment according to Figure 5, longitudinal elastic threads 65 are arranged in a pretensioned state on the inner layer 4 in the two outer edge portions for drawing said covering together along the absorbent crotch element. Transverse elastic threads 66 are arranged in a pretensioned state on the liquid-permeable layer across the crotch element 63. These threads extend outside the crotch element in the lateral direction. During use of the article, by virtue of the action of principally the transverse elastic threads 66, the liquid-impermeable layer 5 will be drawn up around the side edges of the absorbent element. The longitudinal elastic threads 65 contribute to keeping the edge portions of the article at the absorbent crotch element in contact with the skin of the wearer. The elastic threads 65 and 66 therefore contribute to increased protection against leakage in the lateral direction of the article in the crotch area and also provide the absorbent element 63 with increased firmness and dimensional stability.

The second stiffening part element 62 extends with its leg portions 14, 15 in over the rear portion 2. The outer side edges 18, 19 of the second stiffening part element 62 on the legs 14, 15 diverge from the crotch portion in over the rear portion. In a rear transition area 20 between the crotch portion 3 and the rear portion 2, said outer edge sides 18, 19 abruptly change direction in relation to the edge sides 22, 23 of the stiffening element in the crotch portion of the article.

The purpose of the edge sides 18, 19 of the second stiffening part element 62 diverging in the backward direction on the rear portion 2 is that the article, in addition to being anchored firmly at the transition 12 between the front portion and the crotch portion, will also be anchored at the rear in the transition area between the crotch portion 3 and the rear portion 2, as a result of which the article is very stable and well fixed on the wearer during use at the same time as it feels comfortable for the wearer by virtue of its anatomical adaptation in terms of shape, size and geometry. In the drawing, an angle between the longitudinal direction of the article and each outer edge side 18, 19 has been designated by γ. For a good anchoring function, this angle should not be less than roughly 30°. Furthermore, so as not to feel uncomfortable, the angle should not exceed roughly 60°.

The distance G between the transition areas 12 and 20 is adapted to the crotch length of a wearer and, as mentioned above in connection with the embodiments according to Figures 1-4, this distance G is suitably of the order of 70-120 mm. As mentioned above, the essentially plane area of the crotch of women directly in front of the genitals has a length of in the order of 80-100 mm, that is to say all women are essentially the same size in this plane area. It has been found that a crotch dimension G on the article of in the order of 70-120 mm functions well for most wearers. The larger the angles α and γ and the stiffer the stiffening element, the more important it is that the crotch dimension on the article corresponds to the length of the plane crotch portion of the intended wearer directly in front of her genitals if the article is not to feel uncomfortable.

It may therefore be suitable to have a range of sizes of the article according to the invention depending on the selection of stiffness and said angles, so that different wearers can find a suitable size with regard to dimensions and angles. This of course applies to all the embodiments of the invention described here but is particularly important when the article is intended to be anchored both at the front and at the rear. The requirement for size adaptation also increases for all the embodiments the stiffer the stiffening part elements 61 and 62 are.

The second stiffening part element 62 in the embodiment according to Figure 5 has a cutout 13. As in other illustrative embodiments described above, this is wedge-shaped but has a larger angle β which, in Figure 5, is obtuse. The angle β can vary within wide limits between of the order of 10° and 120°. How large a cutout 13 is required depends on the function required of the legs 14 and 15 and the absorption capacity required in the rear portion 2 of the article.

The smaller the angle β, with the same width of the rear portion in its entirety and with the same angle γ, the wider are the legs 14, 15, which in turn results in increased stiffness in the rear portion and also increased absorption capacity if the second part element is absorbent.

The size of the cutout also influences the height of the fold 17. This fold height and the shaping of the rear piece 2 also depend on the pretensioning and the extent of the elastic means 16.

The illustrative embodiment of the article according to the invention shown in Figure 5 can serve as, for example, a night towel. Like other embodiments, this embodiment is also suitable as an incontinence pad. This type of protection should be capable of rapidly receiving large quantities of liquid discharged at a high flow rate from the wearer.

An article of the type shown in Figure 5 can, in combination with supporting pants or with special elastic pants adapted for supporting the article, serve as a nappy for receiving both urine and motions. If the article is to serve as a nappy, the cutout 13 should be relatively large, corresponding on the whole to that shown in Figure 5, in order for it to be possible for discharged motions to be taken up in the cutout 13 of the rear portion.

Directly in front of said cutout 13, the covering of the article, consisting of the inner layer 4 and the outer layer 5, can be designed with a bellows-like fold (not shown) with an opening towards the wearer for receiving motions in the pouch formed by the bellows fold.

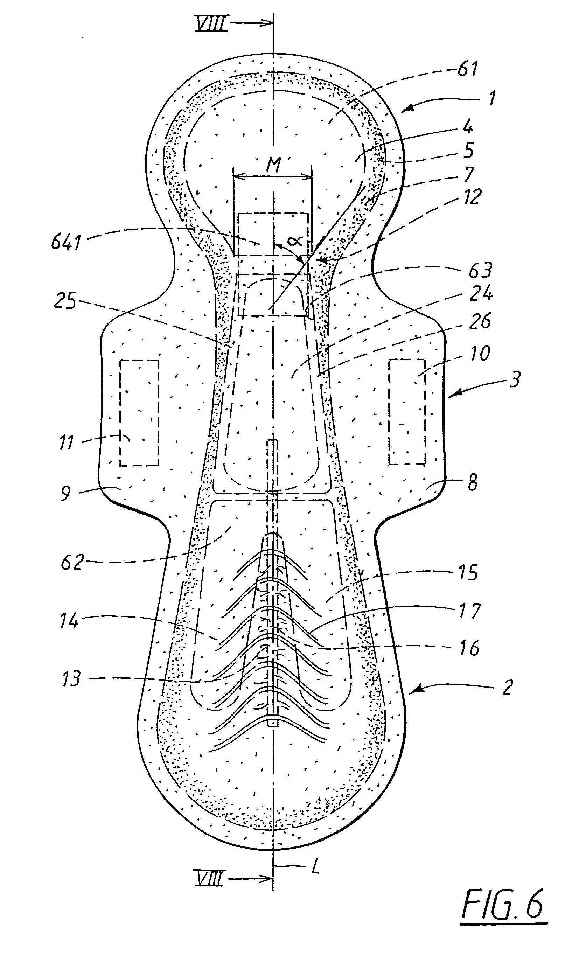

Figures 6-9 show a suitable embodiment of an article according to the invention. This embodiment corresponds in many respects to the embodiments according to Figures 1-4, and those parts corresponding to the same parts in the embodiments described above have been provided with the same reference numbers in the drawing.

In the embodiment according to Figures 6-9, the crotch element 63 is connected to the first part element 61 by means of a short elastic strip 641, as a result of which the crotch element 63 and the first part element can be moved away from one another slightly counter to the action of the elastic strip. The elastic strip can be replaced by elastic bands or threads.

A way of reducing further the risk of edge leakage caused by the sanitary towel being deformed during use, in addition to the arrangement of the stiffening part elements, is to provide the sanitary towel with a raised portion, what is known as a hump, which raised portion has been designated by reference number 240. The raised portion or hump is intended to make contact with the genitals of the wearer during use of the sanitary towel. Discharged bodily fluid can in this way be caught as soon as it leaves the body of the wearer and be absorbed immediately into the article instead of running out over the surface of the latter.

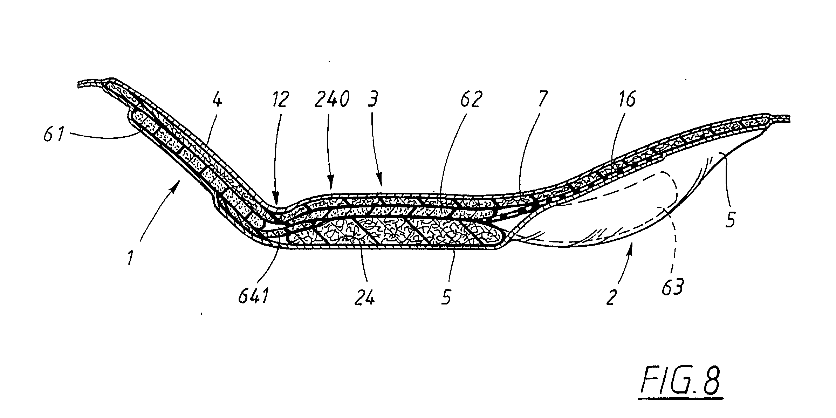

In the embodiment shown in Figures 6-9, the hump is brought about by a hump-forming element 24 which, as can be seen most clearly from Figure 8, is arranged below the absorbent crotch element 63 inside the liquid-impermeable outer layer 5. The positioning of the hump-forming element results in a number of advantages. Admission of bodily fluid is not interfered with by hump material in direct proximity to the genitals of the wearer, but the parts located closest to the genitals of the wearer can be optimized with regard to admission and absorption capacity.

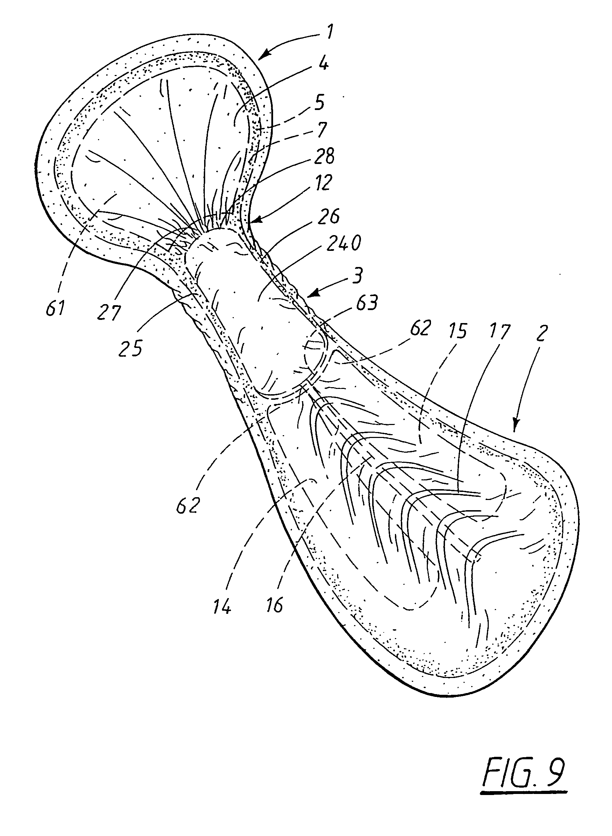

The positioning selected for the hump-forming element below the absorbent crotch element in combination with the positioning along the crotch portion of the article also has the positive effect that the hump-forming element contributes to the article curving and shaping itself in the desired manner when fitted on the wearer. At the transition 12 between the crotch portion 3 and the front portion, as can be seen from Figure 9, a point of inflexion 27 is formed, in front of which, that is to say in the front portion of the article, the article is concave at least over a portion next to said transition 12. Behind said point of inflexion, that is to say along the crotch portion of the article, the article is, in the area directly in front of the hump-forming element 24, convex, that is to say the absorbent crotch element 63 is curved upwards in this area, as can be seen most clearly from Figures 8 and 9.

The hump-forming element 24 consists of, for example, a non-absorbent synthetic wadding which has resilient properties. Such a hump-forming element retains its shape and function even when the material is in a wet state.

The hump-forming element can also consist of a foamed material, for example polyurethane foam or the like.

As the hump-forming material is, in the embodiment shown, located below the absorbent crotch element 63, the hump-forming material can be liquid-absorbing. In such a design, it is suitable to select a material which has larger capillaries than the absorbent crotch element 63 has, so that liquid can be transported to the hump-forming material only when the crotch element is saturated with liquid. A hump-forming absorbent fibrous layer which has resilient properties only in the dry state can therefore also be used in such a construction because the material is essentially dry until the absorption element itself is saturated with liquid. The positioning of the hump-forming element 24 below both the stiffening and the absorbent element therefore affords a number of important advantages.

The element forming the raised portion 240 has an elongate shape and extends over the entire crotch portion in the illustrative embodiment shown. The length of the raised portion can vary between roughly 20 mm and 120 mm.

Superabsorbent material, in the form of particles or fibres for example, can be included in the hump so as to raise it gradually in connection with menstrual fluid or urine being taken up.

The element 24 forming the raised portion is narrower than the rest of the article in the crotch area. In this way, it is made possible for laterally surrounding portions 25, 26 of the rest of the article to shape themselves around the element 24 forming the raised portion. The material forming the raised portion is also suitably thicker than the surrounding areas 25, 26.

In Figure 8, the article has been shown in curved, three-dimensional form for the sake of clarity. An absorbent article of the type described here is of course always three-dimensional in the conventional sense, that is to say it has length, width and thickness.

In this context, however, the term three-dimensional means that the article must be curved in some way to adapt to the body shape of the wearer.

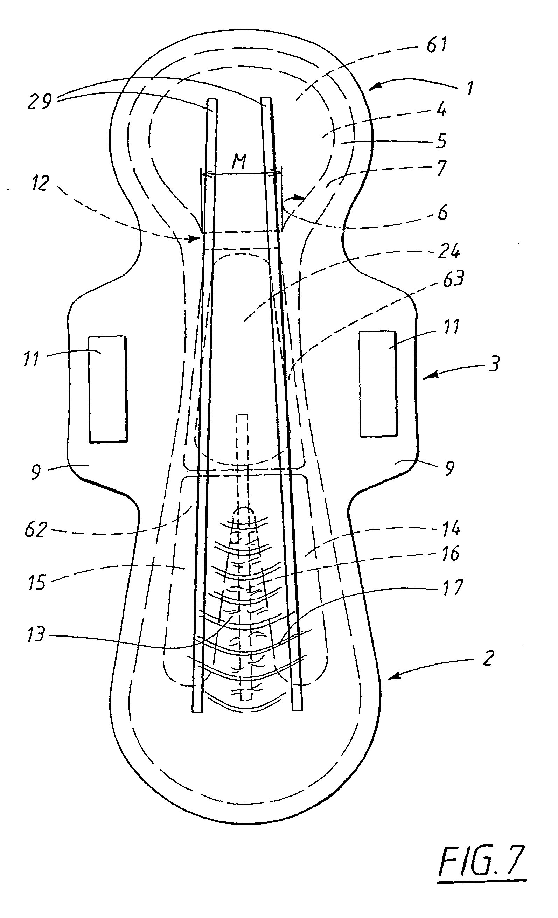

In this context, the term plane form means that the article is essentially plane. The article shown in Figures 6 and 7 is essentially planiform according to this definition in spite of the fact that the elastic means draws the material layers together in the cutout 13 between the legs 14, 15.

Articles in plane form according to Figures 6 and 7 can be packed simply, for example in stacks in a box or bag and yet, when put on, be made to adopt an anatomically adapted three-dimensional shape, as shown in Figures 8 and 9, without any measures whatsoever.

By virtue of its special design with the width of the front stiffening part element exceeding the distance between said muscle tendons, the design of the hump-shaped element 24, the action of the elastic means 16 and the stiffness and geometric shape of the stiffening part elements 61, 62, the article is anatomically adapted and predestined to adopt during handling a three-dimensional shape according to Figures 8 and 9 adapted to the body shape of the wearer.

In the illustrative embodiment shown, the stiffening and also absorbent part elements 61 and 62 have the same stiffness properties over their entire extent. As a result, uncontrolled creases, which could give rise to uncontrolled and unintentional liquid flow, do not normally arise over the extent of the stiffening part elements. At the transition 12 between the crotch portion 3 and the front portion 1, curvature is initiated because the article as a whole changes its flexural resistance here, on account of the first stiffening part element having its end around this transition. The first stiffening part element is narrowest here with a dimension M which can be adapted to the distance between said muscle tendons of the wearer or smaller than said distance. At this transition 12, a point of inflexion 27 is formed, in front of which the article is concave and bowl-shaped, whereas it adopts a convex shape behind this point of inflexion 27. In the embodiment according to Figure 9, the hump-forming element is rounded at the front along a line 28. In this way, the first stiffening part element 61 in the front portion is caused to adopt an evenly rounded bowl shape in the front portion, as can be seen from Figure 9.

In the transition area 20 between the crotch portion 3 and the rear portion 2 as well, the hump-forming element 24, which in the embodiment shown extends as far as said transition area 20, is rounded at its rear end. As a result, no undesirable creases arise, but the transition between the convex crotch portion and the two side portions of the rear portion 2 sloping downwards around the fold 17 formed by the elastic means 16 is even and smooth without undesirable creases.

The raised portion 240 formed by the hump-forming element 24 also has the advantage that the fold extending into the cleft between the buttocks of the wearer does not extend in too abruptly or too far and give rise to chafing. In this respect also, the hump provides a soft transition in the transition area between the crotch portion and the rear portion.

In all the embodiments described above, it is suitable for the article to be provided with a pressure-sensitive adhesive on the outside of its liquid-impermeable outer layer 5. This has been indicated in Figure 7 by adhesive strands 29 which, before use of the article, are covered in a conventional manner by a cover strip 30 treated with release agent. Although the article according to the invention is anatomically adapted, it is suitable, for reliable secure positioning, to have a pressure-sensitive adhesive on the liquid-impermeable outside of the article for interaction with the briefs of the wearer, which contributes to keeping the article in the intended position on the wearer.

According to one embodiment (not shown in the drawing), the article can be attached to or interact with the body of the wearer by means of adhesive or friction coating. The friction means or adhesive can be the only means of attachment, but it can also be used in combination with pressure-sensitive adhesive intended for attachment to the briefs of the wearer.

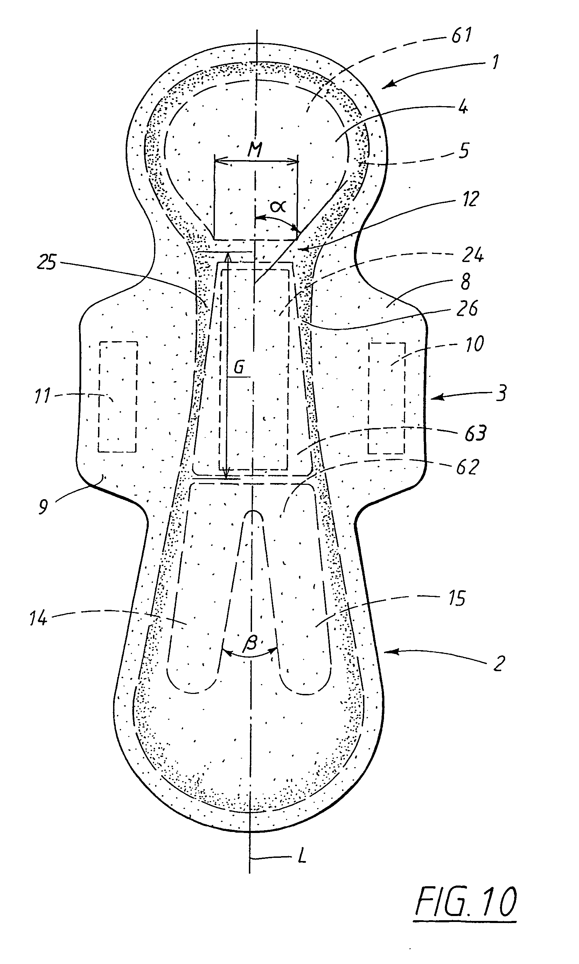

Figure 10 shows an embodiment which is modified slightly in relation to the embodiment according to Figures 6-9. Those parts in the article according to Figure 10 corresponding to similar components in the embodiments according to Figures 6-9 have been provided with the same reference numbers.

The article shown in Figure 10 is simpler in terms of manufacture than the embodiment according to Figures 6-9. The article according to Figure 10 has no longitudinal elastic in the cutout 13 between the legs 14 and 15 of the stiffening part element 62.

During use of an article according to Figure 10, the rear portion 2 is folded along the line L in spite of the absence of the elastic means. In this case also, a stiffening of the rear portion is therefore obtained after folding of the rear portion along the line L. The flexural rigidity increases after the article has been folded along the line L, which results in the rear portion of the article being more stable. During use of the article, the fold formed along the line L will penetrate part of the way into the cleft between the buttocks of the wearer and thus contribute to the article staying in place in the lateral direction at the same time as the fold catches any bodily fluid which runs in the cleft between the buttocks of the wearer.

The article according to Figure 10 also differs from the embodiment according to Figures 6-9 in that the hump-forming element 24 has straight end edge sides and also the same width along its entire length. The hump-forming element is suitably of such a thickness that, directly in front of the raised portion 240, the article is at least twice as thick as the surrounding areas 25, 26.

In all embodiments, the hump-forming element can be slit in the longitudinal direction, as a result of which the hump can expand more easily.

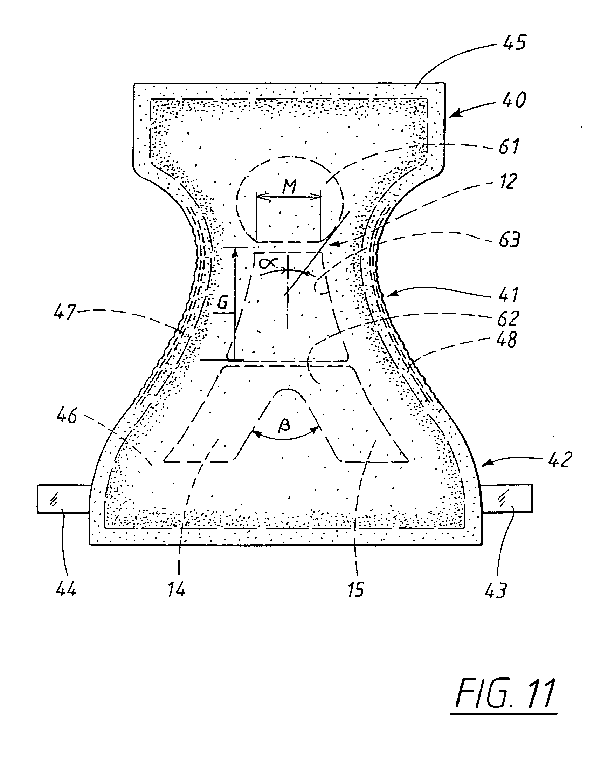

Figure 11 shows an embodiment in the form of a nappy. This has a front portion 40, a crotch portion 41 and a rear portion 42. Outwardly, the embodiment shown of an article in the form of a nappy according to the present invention is of conventional design. When the nappy is put on, the front portion 40 and the rear portion 42 are intended to be fitted around the waist of the wearer and to be closed in the fitted position by means of tape flaps 43, 44. In Figure 11, the nappy is shown diagrammatically in plane form from the inside and is provided with a covering in the form of a liquid-permeable inner layer 45, suitably made of non-woven fabric, and an outer layer made of thin plastic film (not shown), suitably made of polyethylene. Inside the inner layer, an essentially hourglass-shaped unit consisting of stiffening part elements 61, 62 and an absorbent crotch element 63 is indicated. In the crotch portion, leg elastic 47, 48, which is intended to fit tightly around the thighs of the wearer during use of the nappy, has been arranged along the edge portion.

Figure 11 shows diagrammatically stiffening part elements 61, 62 and an absorbent crotch element 63 of the same type as described in the illustrative embodiments described above. In Figure 11, components corresponding to similar parts in illustrative embodiments described above have been provided with the same reference numbers. The stiffening absorbent elements are anatomically adapted in the same way as in illustrative embodiments described above, with a dimension M adapted to the distance between said muscle tendons directly in front of the groins, with a crotch length G adapted to the crotch length of the wearer, and with other angles and geometry as described above. The dimension M in the illustrative embodiment shown is smaller than or the same as said distance between said muscle tendons of the wearer. The essential feature is that the front part element 61 has a width somewhere along the longitudinal direction of the article which exceeds said distance.

As mentioned above, a person has essentially the same dimension between said muscle tendons throughout his or her life. Nappies according to Figure 11 therefore function in principle for both children and adults if the nappy as a whole is adapted in terms of size.

A nappy according to the invention of the type shown in Figure 11 has a superior fit compared with conventional nappies. The presence of the stiffening part elements 61, 62 means that, when the nappy is put on, it is guided into the correct position on the wearer and that it remains in this position during use of the article. The nappy can suitably be provided with transverse elastic (not shown) in the rear portion in order to draw the legs 14, 15 together. The legs can then be connected by only the liquid-permeable layer. The liquidtight layer is then provided with a bellows-shaped fold midway between the legs, which fold has an opening towards the wearer, in addition to which the article has, between the legs, an opening in the liquid-permeable layer for admitting motions into the pouch formed by the bellows fold.

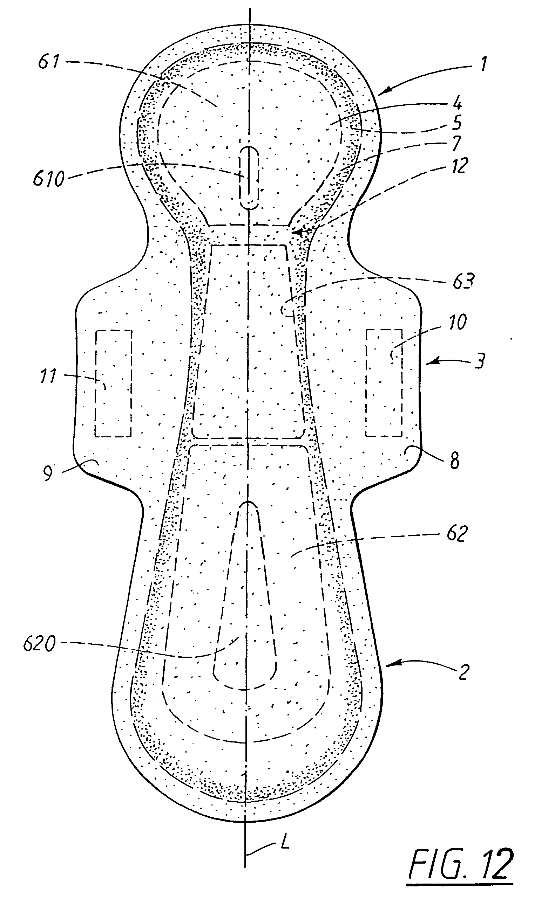

The sixth embodiment of a sanitary towel according to the invention shown in Figure 12 differs from the embodiment described above in connection with Figures 1 and 2 in that the first stiffening part element 61 has been provided with an elongate first through-hole 610 and in that the second stiffening part element 62 has an elongate second through-hole 620 instead of the cutout 13. In Figure 12, those parts corresponding to similar components in other illustrative embodiments have been provided with the same reference numbers. The materials used in the embodiment according to Figure 12 can be of the type described above in connection to other illustrative embodiments.

As can be seen from Figure 12, the first through-hole 610 arranged in the first part element is oblong and extends in the longitudinal direction of the article and along the centre line of the article. The purpose of the first hole is to facilitate curvature of the first part element and also to make possible resilient compression in the lateral direction of the first part element 61 when lateral forces act against the side edges of the first part element.

The second stiffening part element 62 in the rear portion 2 of the article is provided with an elongate second hole 620 which extends in the longitudinal direction of the article and along the longitudinal centre line of the article. During use of the article, lateral forces acting against the second stiffening part element of the article create a fold along the longitudinal direction of the article along said second hole 620. This fold extends into the cleft between the buttocks of the wearer during use and stabilizes the article in position on the wearer. As the second stiffening part element in the embodiment according to Figure 12 has no legs which can move in relation to one another, the elongate hole provides the rear portion of the article with significantly increased dimensional stability in both the lateral and the vertical direction compared with the wedge-shaped cutout in embodiments described above.

In all the illustrative embodiments described above, the width of the absorbent crotch element 63 increases continuously from the transition 12 between the front portion 1 and the crotch portion 3 to the transition area 20 between the crotch portion 3 and the rear portion. One reason for this is that the available space between the legs of the wearer is very limited, and it is important to make optimum use of the width of this area. The width can increase by of the order of 1.5 times between the transition 12 and the transition area 20 without this feeling uncomfortable for the wearer. Another reason is that the article lies more stably on the wearer when the crotch element-is made as wide as possible along the crotch portion.

The invention is not limited to the illustrative embodiments described above, but a large number of modifications are possible within the scope of the patent claims below.

For example, anatomically shaped stiffening and absorbent elements of the type described above can be arranged in what are known as pant nappies, that is to say where the nappy is integrated into disposable pants.

It has been stated above that the stiffening absorbent elements can be made from different materials and from laminates made of one or more material(s). The stiffening part elements can also be made from more than one layer and with the extent of the individual layers being different, in which way it is possible for different areas of the stiffening part elements to have different stiffness.

As mentioned above, the stiffening part elements can consist of all the material layers and bonding agents included. Different stiffness in different areas of the stiffening element can therefore also be obtained by varying the degree of connection in different areas, for example different quantities of adhesive in different areas and even the absence of adhesive or other bonding agent in different areas between or in individual layers.

The elastic means 16, which is arranged in the cutout 13, has been indicated in the illustrative embodiments described above as having been arranged in a pretensioned state. However, in the manufacture of absorbent articles such as sanitary towels, nappies and the like, it is known to arrange a heat-sensitive elastic means in an untensioned state and to tension the elastic by heat treatment. This suitably takes place when the articles are packed.

In the illustrative embodiments described above relating to articles for arrangement inside the crotch portion of briefs, the article is in the majority of the illustrative embodiments provided with permanently arranged wings for attachment of the article to the briefs with the wings felded around the edge portion of the briefs and attached on the outside of the crotch portion. The wings can consist of separate elements which are attached to the rest of the article in connection with the article being put on. The separate wings can be arranged detachably on the rest of the article during manufacture of the article, as a result of which a wearer who does not want to have wings on the article can remove these in connection with putting the article on.

The illustrative embodiments described above which do not have wings can be provided with separate wings either during manufacture or when the article is put on.

## Claims

1. Absorbent article, such as a sanitary towel, a panty liner, an incontinence pad, a nappy or the like, which article has a longitudinal direction and a transverse direction, a front portion (1), a rear portion (2), a crotch portion (3) located between the rear portion and the front portion, an absorbent element and a liquidtight layer (5), and also a stiffening area (6) which is intended to contribute to the three-dimensional shape of the article during its use, **characterized in that** the stiffening area (6) is in a plane state before use of the article and comprises a first stiffening part area (61) in the front portion (1), **in that** the first stiffening part area (61) in the front portion (1) has a width which, at least at some point in the longitudinal direction of the article, exceeds the distance between the muscle tendons of the wearer on both sides of the crotch of the wearer in the groin of the latter, which distance is about 25-45 mm, **in that** the article comprises an elongate absorbent crotch area (63) which is intended to be fitted in the crotch of the wearer over the genitals of the latter and is designed for an optimum absorption function by free selection of absorption material and construction, **in that** the absorbent crotch area (63) is attached to the first part area (61) and held in place by the latter so as to avoid the absorbent crotch area sliding backwards and out of the intended position over the genitals of the wearer when the wearer moves in that in the front portion (1) of the article, the side edges of the first stiffening part area (61) diverge in the forward direction from the crotch portion (3) in over the front portion (1), **In that**, in the direction from the crotch area, the side edges of the stiffening part element (61) form an acute angle (α) with a line in the longitudinal direction of the article and **in that** the first stiffening part area (61) extends into the transition between the front portion and the crotch portion of the article, and **in that** the first stiffening part area (61) has a width (M) at said transition of in the order of 20-35 mm.

2. Article according to Claim 1, **characterized in that** the absorbent crotch area (63) is less stiff than the first stiffening part element (61).

3. Article according to Claim 1 or 2, **characterized in that,** in relation to said stiffening area, said absorbent crotch area is soft and yielding when utilization stresses occur.

4. Article according to any one of the preceding claims, **characterized in that** the absorbent crotch area (63) consists of a material which is different from that in the first part area.

5. Article according to any one of the preceding claims, **characterized in that** the absorbent crotch area (63) comprises or consists of an absorbent foamed material.

6. Article according to any one of Claims 1-3, **characterized in that** the absorbent crotch area (63) comprises or consists of cellulose fluff pulp.

7. Article according to any one of the preceding claims, **characterized in that** the absorbent crotch area (63) comprises superabsorbent material which is mixed with a material forming part of the crotch area, such as fluff pulp or absorbent foam, or which is arranged in one or more plies in the absorbent crotch area.

8. Article according to Claim 1, **characterized in that** said width (M) of the first stiffening part area (61) at the transition between the crotch portion (3) and the front portion (1) is of the order of 25-30 mm.

9. Article according to any one of the preceding claims, **characterized in that** a second stiffening part area (62) extends part of the way in over the rear portion (2) of the article, and **in that** the side edges (18. 19) of the second stiffening part area (62) diverge in the direction from the crotch portion (3) at least part of the way from the crotch portion in over the rear portion of the article.

10. Article according to Claim 9, **characterized in that** the second stiffening part area (62) has a cutout (13) extending from its end edge in the rear portion (2) in the direction towards the crotch portion (3), as a result of which the article is during use provided with a fold (17) along the longitudinal direction of the article in said cutout, which fold extends into the cleft between the buttocks of the wearer during use of the article.

11. Article according to Claim 9, **characterized in that** the second stiffening part area (62) has an elongate second through-hole (620) which extends in the longitudinal direction of the article and along the centre line of the article, as a result of which the article is during use provided with a fold along the longitudinal direction of the article along said second hole (620), which fold extends into the cleft between the buttocks of the wearer during use of the article and thus stabilizes the article in position on the wearer.

12. Article according to Claim 11, **characterized in that** said cutout (13) is wedge-shaped and located symmetrically and forms an angle (β) of between 10 and 120°, preferably between 15 and 40°, at its end facing the crotch portion.

13. Article according to any one of Claims 8-12, **characterize in that** the second part area (62) is made from the same material as the first part area (61).

14. Article according to any one of Claims 8-13, **characterized in that** the second part element (62) is made with the same stiffness as the first part element (61).

15. Article according to any one of the preceding claims, **characterized in that** the first stiffening part element (61) has a stiffness in the dry state of in the order of 1-15 N measured according to ASTM D 4032-82.

16. Article according to any one of the preceding claims, **characterized in that** said stiffening part areas (61, 62) consist of compressed part areas of a continuous material body made in one piece, which forms said absorbent crotch area (63) between said part areas.

17. Article according to any one of the preceding claims, **characterized in that** said stiffening part areas (61, 62) consist of part areas of a body made in one piece from foamed material, which part areas are compressed and bonded in compressed state.

18. Article according to any one of Claims 1-16, **characterized in that** at least one of said stiffening part areas consists of a separate part element.

19. Article according to Claim 18, **characterized in that** said stiffening part area (61, 62) also serves as an absorbent element and consists of a number of interconnected tissue layers with, arranged between the layers, superabsorbent material in the form of particles or fibres.

20. Article according to any one of Claims 1-16, **characterized in that** the first stiffening part area (61) consists of a dry-formed fibre mat with a density between 0.15 and 0.75 g/cm³ and a weight per unit area of in the order of 100-400 g/m².

21. Article according to Claim 20, **characterized in that** the dry-formed fibre mat is, after compression, mechanically softened to the desired stiffness.

22. Article according to Claim 20 or 21, **characterized in that** the dry-formed fibre mat is provided with the desired reduced stiffness and the desired extensibility by virtue of the degree of compression selected and the compression pattern selected.

23. Article according to any one of the preceding claims, **characterized in that** the first stiffening part area (61) has a stiffness of at least 1.0 N, and **in that** the first stiffening part area is designed with essentially the same stiffness over the entire extent of the stiffening part area.

24. Article according to any one of the preceding claims, **characterized in that** the side edges of the first stiffening part element (61), which diverge at least part of the way from the crotch portion (3) in over the front portion (1) of the article, are arranged so as to form an angle (α) between a line in the longitudinal direction of the article and each of said side edges of in the order of 35-55°, preferably in the order of 45°.

25. Article according to any one of Claims 8-24, **characterized in that** the absorbent crotch element (63) is attached to the second stiffening part area (62) and held in place by the latter so as to avoid the article sliding forwards when the wearer moves.

26. Article according to any one of the preceding claims, **characterized in that** said attachment of the first stiffening part area to the crotch area consists of a connection via a connection element forming part of the article.

27. Article according to Claim 26, **characterized in that** said connection element consists of the liquidtight layer (5).

28. Article according to Claim 26, **characterized in that** said connection element consists of an elastically stretchable element (64), as a result of which the crotch area (63) can move relative to the first part area (61) and, by virtue of the action of the elastic, tends to return to its original position.

29. Article according to any one of Claims 25-28, **characterized in that** said attachment of the second stiffening part area (62) to the crotch area consists of a connection via a connection element forming part of the article.

30. Article according to Claim 29, **characterized in that** said connection element consists of the liquidtight layer (5).

31. Article according to Claim 28, **characterized in that** said connection element consists of an elastically stretchable element, as a result of which the crotch area can move relative to the second part area and, by virtue of the action of the elastic, tends to return to its original position,

32. Article according to any one of the preceding claims, **characterized in that** a hump-forming element (24) made of a resilient material is arranged under the absorbent crotch area (63) over at least part thereof, which hump-forming element is arranged so as to bring about a raised portion (240) on the side which is intended to be fitted against the wearer, the raised portion being arranged so as to come to lie directly in front of the genitals of the wearer after fitting of the article on the wearer.

33. Article according to Claim 32, **characterized in that** the raised portion (240) is elongate in the longitudinal direction of the article and has a length of between 20 mm and 120 mm.

34. Article according to Claim 32 or 33, **characterized in that** the raised portion (240) is narrower than the rest of the article in the crotch area, and **in that** the raised portion is thicker than the surrounding areas.

35. Article according to any one of the preceding claims, **characterized in that** an elastic, means (16) is arranged in the longitudinal direction of the article and centrally along the rear portion (2) of the article and along at least part thereof from the crotch portion (3), which elastic means is intended, along its length, to draw adjacent material portions together and curve the article upwards for better contact with the body of the wearer.

36. Article according to any one of the preceding claims, **characterized in that** the first stiffening part area (61) also serves as an absorption element and is essentially homogeneous over its entire extent with regard to thickness, stiffness, spreading capacity and absorption capacity, as a result of which the first part area curves evenly during use of the article without forming local irregularities which may give rise to undesirable spreading of liquid.

37. Article according to any one of the preceding claims, **characterized in that** the second stiffening part area (62) also serves as an absorption element and is essentially homogeneous over its entire extent with regard to thickness, stiffness, spreading capacity and absorption capacity, as a result of which the second part area curves evenly during use of the article without forming local irregularities which may give rise to undesirable spreading of liquid.

38. Article according to any one of the preceding claims, **characterized in that** the width of the crotch area (63) behind said transition area (12) increases continuously in the crotch portion (3) in the backward direction towards the rear portion (2) for the purpose of optimally utilizing available width space in this area with regard to maximum absorption.

39. Article according to any one of the preceding claims, **characterized in that** the article is arranged so as, by virtue of the stiffness selected for the first stiffening part area (61) and by virtue of the selection of geometry and dimensions in and around the transition (12) between the crotch portion (3) and the front portion (1), when the article is positioned in connection with it being put on with the transition between the front portion and the crotch portion between said muscle tendons, to be fixed in between these and in this way be transformed from plane form to three-dimensional form with the front portion curved upwards in relation to the crotch portion and forming a bowl-like shape at least in an area next to the crotch portion.

40. Article according to any one of the preceding claims, **characterized in that** the first stiffening part area (61) has a first through-hole (610) which is oblong and extends in the longitudinal direction of the article and along the centre line of the article, and which makes possible resilient compression in the lateral direction of the first part area when lateral forces act against the side edges of the first part area.

41. Article according to any one of the preceding claims, **characterized in that** the absorbent crotch area (63) has a length (G) of in the order of 70-120 mm.

42. Article according to Claim 10, **characterized in that** the article consists of a nappy, **in that** elastic which is transverse in relation to the longitudinal direction of the article is arranged in the rear portion across said cutout (13), and **in that** the transverse elastic is arranged so as to draw together the, covering material of the article in said cutout, as a result of which an expandable pocket intended for receiving motions is formed in said cutout (13).

## Patentansprüche

1. Absorptionsartikel, wie zum Beispiel eine Binde, eine Slipeinlage, ein Inkontinenzbelag, eine Windel oder dergleichen, welcher Artikel eine Längsrichtung und eine Querrichtung, einen Frontabschnitt (1), einen Rückabschnitt (2), einen Schrittabschnitt (3), der zwischen dem Rückabschnitt und dem Frontabschnitt angeordnet ist, ein Absorptionselement und eine flüssigkeitsdichte Lage (5) und auch einen Versteifungsbereich (6) enthält, der dafür gedacht ist, zu der dreidimensionalen Form des Artikels während seiner Benutzung beizutragen, **dadurch gekennzeichnet, dass** der Versteifungsbereich (6) vor der Verwendung des Artikels in einem ebenen Zustand ist und einen ersten Versteifungsteilbereich (61) in dem Frontabschnitt (1) umfasst, dass der erste Versteifungsteilbereich (61) in dem Frontabschnitt (1) eine Breite aufweist, die zumindest an einem Punkt in der Längsrichtung des Artikels über den Abstand zwischen den Muskelsehnen des Trägers an beiden Seiten des Schritts des Trägers in der Leistengegend des letztgenannten hinausgeht, welcher Abstand etwa 25-45 mm ist, dass der Artikel einen länglichen Absorptionsschrittbereich (63) umfasst, der dazu gedacht ist, in dem Schritt des Trägers über dien Genitalien des letztgenannten angebracht zu werden und für eine optimale Absorptionsfunktion durch freie Auswahl von Absorptionsmaterial und Konstruktion gestaltet ist, dass der Absorptionsschrittbereich (63) an dem ersten Teilbereich (61) angebracht und durch den letztgenannten am Ort gehalten wird, um zu verhindern, dass der Absorptionsschrittbereich nach hinten und aus der vorgesehenen Position über den Genitalien des Trägers rutscht, wenn sich der Träger bewegt, dass die Seitenkanten des ersten Versteifungsteilbereichs (61) in dem Frontabschnitt (1) des Artikels in der Vorwärtsrichtung von dem Schrittabschnitt (3) über den Frontabschnitt (1) divergieren, dass die Seitenkanten des Versteifungsteilelements (61) in Richtung von dem Schrittbereich einen spitzen Winkel (α) mit einer Linie in der Längsrichtung des Artikels bilden und dass sich der erste Versteifungsteilbereich (61) in den Übergang zwischen dem Frontabschnitt und dem Schrittabschnitt des Artikels erstreckt und dass der erste Versteifungsteilbereich (61) eine Breite (M) an dem Übergang von in der Größenordnung von 20-35 mm aufweist.

2. Artikel nach Anspruch 1, **dadurch gekennzeichnet, dass** der Absorptionsschrittbereich (63) weniger steif als das erste Versteifungsteilelement (61) ist.

3. Artikel nach Anspruch 1 oder 2, **dadurch gekennzeichnet, dass** der Absorptionsschrittbereich in Relation zu dem Versteifungsbereich weich und nachgiebig ist, wenn Benutzungsspannungen auftreten.

4. Artikel nach einem der vorhergehenden Ansprüche, **dadurch gekennzeichnet, dass** der Absorptionsschrittbereich (63) aus einem Material besteht, das verschieden von dem in dem ersten Teilbereich ist.

5. Artikel nach einem der vorhergehenden Ansprüche, **dadurch gekennzeichnet, dass** der Absorptionsschrittbereich (63) ein absorbierendes geschäumtes Material umfasst oder daraus besteht.

6. Artikel nach einem der Ansprüche 1-3, **dadurch gekennzeichnet, dass** der Absorptionsschrittbereich (63) Zelluloseflockenzellstoff umfasst oder daraus besteht.

7. Artikel nach einem der vorhergehenden Ansprüche, **dadurch gekennzeichnet, dass** der Absorptionsschrittbereich (63) ein superabsorbierendes Material umfasst, das mit einem ein Material bildenden Teil des Schrittbereichs, wie zum Beispiel Flockenzellstoff oder Absorptionsschaum, gemischt ist oder das in einer oder mehreren Stofflagen in dem Absorptionsschrittbereich angeordnet ist.

8. Artikel nach Anspruch 1, **dadurch gekennzeichnet, dass** die Breite (M) des ersten Versteifungsteilbereichs (61) am Übergang zwischen dem Schrittabschnitt (3) und dem Frontabschnitt (1) in der Größenordnung von 25-30 mm ist.

9. Artikel nach einem der vorhergehenden Ansprüche, **dadurch gekennzeichnet, dass** sich ein zweiter Versteifungsteilbereich (62) über einen Teil des Wegs über den Rückabschnitt (2) des Artikels erstreckt und dass die Seitenkanten (18, 19) des zweiten Versteifungsteilbereichs (62) in Richtung von dem Schrittabschnitt (3) zumindest einen Teil des Wegs von dem Schrittabschnitt über den Rückabschnitt des Artikels divergieren.

10. Artikel nach Anspruch 9, **dadurch gekennzeichnet, dass** der zweite Versteifungsteilbereich (62) einen Ausschnitt (13) aufweist, der sich von seiner Endkante in dem Rückabschnitt (2) in der Richtung auf den Schrittabschnitt (3) zu erstreckt, so dass der Artikel als Folge davon während der Benutzung mit einer Falz (17) entlang der Längsrichtung des Artikels in dem Ausschnitt versehen ist, welche Falz sich in die Spalte zwischen den Gesäßhälften des Trägers während der Benutzung des Artikels erstreckt.

11. Artikel nach Anspruch 9, **dadurch gekennzeichnet, dass** der zweite Versteifungsteilbereich (62) eine längliche zweite Durchgangsöffnung (620) aufweist, die sich in der Längsrichtung des Artikels und entlang der Mittellinie des Artikels erstreckt, so dass der Artikel als Folge davon während der Benutzung mit einer Falz entlang der Längsrichtung des Artikels entlang der zweiten Öffnung (620) versehen ist, welche Falz sich in den Spalt zwischen den Gesäßhälften des Benutzers während der Benutzung des Artikels erstreckt und damit den Artikel beim Benutzer in Position stabilisiert.

12. Artikel nach Anspruch 11, **dadurch gekennzeichnet, dass** der Ausschnitt (13) keilförmig und symmetrisch angeordnet ist und einen Winkel (β) von zwischen 10 und 120°, bevorzugt zwischen 15 und 40° an seinem Ende, das dem Schrittabschnitt zugewandt ist, bildet.

13. Artikel nach einem der Ansprüche 8-12, **dadurch gekennzeichnet, dass** der zweite Teilbereich (62) aus demselben Material wie der erste Teilbereich (61) gemacht ist.

14. Artikel nach einem der Ansprüche 8-13, **dadurch gekennzeichnet, dass** das zweite Teilelement (62) mit derselben Steifigkeit wie das erste Teilelement (61) ausgeführt ist.

15. Artikel nach einem der vorhergehenden Ansprüche, **dadurch gekennzeichnet, dass** das erste Versteifungsteilelement (61) eine Steifigkeit in trockenem Zustand in der Größenordnung von 1-15 N, gemessen nach ASTM D 4032-82, aufweist.

16. Artikel nach einem der vorhergehenden Ansprüche, **dadurch gekennzeichnet, dass** die Versteifungsteilbereiche (61, 62) aus komprimierten Teilbereichen eines durchgehenden Materialkörpers, der in einem Stück ausgeführt ist, der den absorbierenden Schrittbereich (63) zwischen den Teilbereichen bildet, bestehen.

17. Artikel nach einem der vorhergehenden Ansprüche, **dadurch gekennzeichnet, dass** die Versteifungsteilbereiche (61, 62) aus Teilbereichen eines Körpers, der in einem Stück aus geschäumtem Material hergestellt ist, bestehen, welche Teilbereiche in komprimiertem Zustand komprimiert und gebunden sind.

18. Artikel nach einem der Ansprüche 1-16, **dadurch gekennzeichnet, dass** zumindest einer der Versteifungsteilbereiche aus einem separaten Teilelement besteht.

19. Artikel nach Anspruch 18, **dadurch gekennzeichnet, dass** der Versteifungsteilbereich (61, 62) auch als ein Absorptionselement dient und aus einer Anzahl miteinander verbundener Gewebelagen besteht, wobei zwischen den Lagen superabsorbierendes Material in der Form von Partikeln oder Fasern angeordnet ist.

20. Artikel nach einem der Ansprüche 1-16, **dadurch gekennzeichnet, dass** der erste Versteifungsteilbereich (61) aus einer trocken geformten Fasermatte mit einer Dichte zwischen 0,15 und 0,75 g/cm³ und einem Gewicht pro Einheitsfläche von in der Größenordnung von 100-400 g/m² besteht.

21. Artikel nach Anspruch 20, **dadurch gekennzeichnet, dass** die trocken geformte Fasermatte nach Kompression mechanisch bis zur gewünschten Steifigkeit aufgeweicht wird.

22. Artikel nach Anspruch 20 oder 21, **dadurch gekennzeichnet, dass** die trocken geformte Fasermatte durch den ausgewählten Kompressionsgrad und das ausgewählte Kompressionsmuster mit der gewünschten reduzierten Steifigkeit und der gewünschten Dehnbarkeit versehen ist.

23. Artikel nach einem der vorhergehenden Ansprüche, **dadurch gekennzeichnet, dass** der erste Versteifungsteilbereich (61) eine Steifigkeit von mindestens 1,0 N aufweist und dass der erste Versteifungsteilbereich mit im Wesentlichen derselben Steifigkeit über die gesamte Ausdehnung des Versteifungsteilbereichs gestaltet ist.

24. Artikel nach einem der vorhergehenden Ansprüche, **dadurch gekennzeichnet, dass** die Seitenkanten des ersten Versteifungsteilelements (61), die zumindest auf einem Teil des Wegs von dem Schrittabschnitt (3) über den Frontabschnitt (1) des Artikels divergieren, so angeordnet sind, dass sie einen Winkel (α) zwischen einer Linie in der Längsrichtung des Artikels und jeder der Seitenkanten von in der Größenordnung von 35-55°, bevorzugt in der Größenordnung von 45° bilden.

25. Artikel nach einem der Ansprüche 8-24, **dadurch gekennzeichnet, dass** das absorbierende Schrittelement (63) an dem zweiten Versteifungsteilbereich (62) angebracht und durch letzteres am Platz gehalten wird, um zu verhindern, dass der Artikel nach vorne rutscht, wenn sich der Träger bewegt.

26. Artikel nach einem der vorhergehenden Ansprüche, **dadurch gekennzeichnet, dass** die Anbringung des ersten Versteifungsteilbereichs an dem Schrittbereich aus einer Verbindung über ein Verbindungselement, das einen Teil des Artikels bildet, besteht.

27. Artikel nach Anspruch 26, **dadurch gekennzeichnet, dass** das Verbindungselement aus der flüssigkeitsdichten Lage (5) besteht.

28. Artikel nach Anspruch 26, **dadurch gekennzeichnet, dass** das Verbindungselement aus einem elastisch dehnbaren Element (64) besteht, so dass der Schrittbereich (63) sich als Folge relativ zu dem ersten Teilbereich (61) bewegen kann und als Folge der Wirkung der Elastik dazu neigt, in seine ursprüngliche Position zurückzukehren.

29. Artikel nach einem der Ansprüche 25-28, **dadurch gekennzeichnet, dass** die Anbringung des zweiten Versteifungsteilbereichs (62) an dem Schrittbereich aus einer Verbindung über ein Verbindungselement besteht, das Teil des Artikels bildet.

30. Artikel nach Anspruch 29, **dadurch gekennzeichnet, dass** das Verbindungselement aus der flüssigkeitsdichten Lage (5) besteht.

31. Artikel nach Anspruch 28, **dadurch gekennzeichnet, dass** das Verbindungselement aus einem elastisch dehnbaren Element besteht, so dass sich der Schrittbereich als Folge relativ zu dem zweiten Teilbereich bewegen kann und als Folge der Wirkung der Elastik dazu neigt, in seine ursprüngliche Position zurückzukehren.

32. Artikel nach einem der vorhergehenden Ansprüche, **dadurch gekennzeichnet, dass** ein eine Aufwölbung bildendes Element (24), das aus einem elastischen Material gemacht ist, unter dem absorbierenden Schrittbereich (63) über zumindest einen Teil davon angeordnet ist, welches die Aufwölbung bildende Element angeordnet ist, um einen angehobenen Abschnitt (240) an der Seite zu erzeugen, die dazu gedacht ist, gegen den Träger angelegt zu sein, wobei der angehobene Abschnitt angeordnet ist, um nach dem Befestigen des Artikels an dem Träger unmittelbar vor den Genitalien des Trägers zu liegen zu kommen.

33. Artikel nach Anspruch 32, **dadurch gekennzeichnet, dass** der angehobene Abschnitt (240) in der Längsrichtung des Artikels länglich ist und eine Länge von zwischen 20 mm und 120 mm aufweist.

34. Artikel nach Anspruch 32 oder 33, **dadurch gekennzeichnet, dass** der angehobene Abschnitt (240) schmaler als der Rest des Artikels in dem Schrittbereich ist und dass der angehobene Abschnitt dicker als die umgebenden Bereiche ist.

35. Artikel nach einem der vorhergehenden Ansprüche, **dadurch gekennzeichnet, dass** ein elastisches Mittel (15) in der Längsrichtung des Artikels und zentral entlang des Rückabschnitts (2) des Artikels und entlang zumindest eines Teils davon von dem Schrittabschnitt (3) angeordnet ist, welches elastische Mittel dazu gedacht ist, benachbarte Materialabschnitte entlang seiner Länge zusammenzuziehen und den Artikel für einen besseren Kontakt mit dem Körper des Trägers nach oben zu krümmen.

36. Artikel nach einem der vorhergehenden Ansprüche, **dadurch gekennzeichnet, dass** der erste Versteifungsteilbereich (61) auch als ein Absorptionselement dient und im Wesentlichen über seine gesamte Ausdehnung in Bezug auf Dicke, Steifigkeit, Streukapazität und Absorptionskapazität homogen ist, so dass sich der erste Teilbereich als Folge während der Benutzung des Artikels gleichmäßig krümmt, ohne lokale Unregelmäßigkeiten zu bilden, die eine ungewünschten Verteilung von Flüssigkeit hervorrufen können.

37. Artikel nach einem der vorhergehenden Ansprüche, **dadurch gekennzeichnet, dass** der zweite Versteifungsteilbereich (62) auch als ein Absorptionselement dient und im Wesentlichen über seine gesamte Ausdehnung in Bezug auf Dicke, Steifigkeit, Streukapazität und Absorptionskapazität homogen ist, so dass sich der zweite Teilbereich als Folge während der Benutzung des Artikels gleichmäßig krümmt, ohne lokale Unregelmäßigkeiten zu bilden, die eine ungewünschte Verteilung von Flüssigkeit hervorrufen können.

38. Artikel nach einem der vorhergehenden Ansprüche, **dadurch gekennzeichnet, dass** sich die Breite des Schrittbereichs (63) hinter dem Übergangsbereich (12) kontinuierlich in dem Schrittbereich (3) in der rückwärtigen Richtung in Richtung des Rückabschnitts (2) zum Zweck des optimalen Verwendens des zu Verfügung stehenden Raums in Breitenrichtung in diesem Bereich in Bezug auf maximale Absorption vergrößert.

39. Artikel nach einem der vorhergehenden Ansprüche, **dadurch gekennzeichnet, dass** der Artikel so angeordnet ist, dass er durch die Steifigkeit, die für den ersten Versteifungsteilbereich (61) ausgewählt ist, und durch die Auswahl der Geometrie und Dimensionen in und um den Übergang (12) zwischen dem Schrittabschnitt (3) und dem Frontabschnitt (1), wenn der Artikel in Verbindung damit positioniert ist, mit dem Übergang zwischen dem Frontabschnitt und dem Schrittabschnitt zwischen den Muskelsehnen angelegt ist, um zwischen diesen befestigt zu sein und auf diese Weise von einer ebenen Form zu einer dreidimensionalen Form überführt zu werden, wobei der Frontabschnitt in Bezug auf den Schrittabschnitt nach oben gekrümmt ist und eine wannenförmige Form zumindest in einem Bereich neben dem Schrittabschnitt bildet.

40. Artikel nach einem der vorhergehenden Ansprüche, **dadurch gekennzeichnet, dass** der erste Versteifungsteilbereich (61) eine erste Durchgangsöffnung (610) aufweist, die länglich ist und sich in der Längsrichtung des Artikels und entlang der Mittellinie des Artikels erstreckt und die eine elastische Kompression in der Seitenrichtung des ersten Teilbereichs ermöglicht, wenn seitliche Kräfte gegen die Seitenkanten des ersten Teilbereichs wirken.

41. Artikel nach einem der vorhergehenden Ansprüche, **dadurch gekennzeichnet, dass** der absorbierende Schrittbereich (63) eine Länge (G) von in der Größenordnung von 70-120 mm hat.

42. Artikel nach Anspruch 10, **dadurch gekennzeichnet, dass** der Artikel aus einer Windel besteht, dass eine Elastik, die in Bezug auf die Längsrichtung des Artikels quer verläuft, in dem Rückabschnitt über den Ausschnitt (13) angeordnet ist und dass die quer verlaufende Elastik angeordnet ist, um das Abdeckmaterial des Artikels in dem Ausschnitt zusammenzuziehen, so dass als Folge eine ausdehnbare Tasche, die dazu gedacht ist, Stuhl aufzunehmen, in dem Ausschnitt (13) ausgebildet wird.

## Revendications

1. Article absorbant, tel qu'une serviette hygiénique, un protège-slip, un tampon contre l'incontinence, une couche ou un article similaire, lequel article présente une direction longitudinale et une direction transversale, une partie avant (1), une partie arrière (2), une partie entrejambe (3) située entre la partie arrière et la partie avant, un élément absorbant et une couche (5) étanche aux liquides, ainsi qu'une zone de raidissement (6) qui est destinée à contribuer à conférer à l'article une forme tridimensionnelle en cours d'utilisation, **caractérisé en ce que** la zone de raidissement (6) se trouve à l'état plan avant l'utilisation de l'article et qu'elle comprend une première zone partielle de raidissement (61) située dans la partie avant (1), **en ce que** la première zone partielle de raidissement (61) située dans la partie avant (1) a une largeur qui, au moins en un certain point dans la direction longitudinale de l'article, excède la distance entre les tendons musculaires de la personne utilisatrice des deux côtés de l'entrejambe de la personne utilisatrice dans l'aine de cette dernière, laquelle distance est d'environ 25 à 45 mm, **en ce que** l'article comprend une zone d'entrejambe absorbante oblongue (63) qui est destinée à s'adapter dans l'entrejambe de la personne utilisatrice sur les parties génitales de celle-ci et qui est conçue en vue d'une fonction d'absorption optimum grâce à une sélection libre du matériau d'absorption et de la structure, **en ce que** la zone d'entrejambe absorbante (63) est fixée à la première zone partielle (61) et est maintenue en place par cette dernière de manière à éviter que la zone d'entrejambe absorbante glisse vers l'arrière et quitte la position souhaitée sur les parties génitales de la personne utilisatrice lorsque la personne utilisatrice se déplace, **en ce que** dans la partie avant (1) de l'article, les bords latéraux de la première zone partielle de raidissement (61) divergent en direction de l'avant depuis la partie entrejambe (3) sur l'étendue de la partie avant (1), **en ce que**, dans la direction partant de la zone d'entrejambe, les bords latéraux de l'élément partiel de raidissement (61) forment un angle aigu (α) avec une ligne s'étendant dans la direction longitudinale de l'article et **en ce que** la première zone partielle de raidissement (61) s'étend dans la transition entre la partie avant et la partie entrejambe de l'article, et **en ce que** la première zone partielle de raidissement (61) a une largeur (M) au niveau de ladite transition, qui est de l'ordre de 20 à 35 mm.

2. Article selon la revendication 1, **caractérisé en ce que** la zone d'entrejambe absorbante (63) est moins rigide que le premier élément partiel de raidissement (61).

3. Article selon la revendication 1 ou 2, **caractérisé en ce que**, par rapport à ladite zone de raidissement, ladite zone d'entrejambe absorbante est souple et se prête lorsqu'il se produit des contraintes d'utilisation.

4. Article selon l'une quelconque des revendications qui précèdent, **caractérisé en ce que** la zone d'entrejambe absorbante (63) est constituée d'un matériau différent de celui de la première zone partielle.

5. Article selon l'une quelconque des revendications qui précèdent, **caractérisé en ce que** la zone d'entrejambe absorbante (63) comprend un matériau absorbant du type mousse, ou bien est constituée d'un matériau absorbant du type mousse.

6. Article selon l'une quelconque des revendications 1 à 3, **caractérisé en ce que** la zone d'entrejambe absorbante (63) comprend de la pâte de fluff de cellulose, ou bien est constituée de pâte de fluff de cellulose.

7. Article selon l'une quelconque des revendications qui précèdent, **caractérisé en ce que** la zone d'entrejambe absorbante (63) comprend un matériau superabsorbant qui est mélangé à un matériau faisant partie de la zone d'entrejambe, tel que de la pâte de fluff ou de la mousse absorbante ou qui est disposé en une ou plusieurs couches dans la zone d'entrejambe absorbante.

8. Article selon la revendication 1, **caractérisé en ce que** ladite largeur (M) de la première zone partielle de raidissement (61) au niveau de la transition entre la partie entrejambe (3) et la partie avant (1) est de l'ordre de 25 à 30 mm.

9. Article selon l'une quelconque des revendications qui précèdent, **caractérisé en ce qu'**une deuxième zone partielle de raidissement (62) s'étend sur une partie de l'étendue de la partie arrière (2) de l'article, et **en ce que** les bords latéraux (18, 19) de la deuxième zone partielle de raidissement (62) divergent dans la direction partant de la partie entrejambe (3) depuis la partie entrejambe sur au moins une portion de l'étendue de la partie arrière de l'article.

10. Article selon la revendication 9, **caractérisé en ce que** la deuxième zone partielle de raidissement (62) comporte une échancrure (13) qui s'étend depuis son bord d'extrémité dans la partie arrière (2) en la direction de la partie entrejambe (3), en conséquence de quoi l'article est muni, lors de l'utilisation, d'un pli (17) s'étendant dans la direction longitudinale de l'article dans ladite échancrure, lequel pli s'étend dans la rainure entre les fesses de la personne utilisatrice pendant l'utilisation de l'article.

11. Article selon la revendication 9, **caractérisé en ce que** la deuxième zone partielle de raidissement (62) comporte un deuxième trou traversant oblong (620) qui s'étend dans la direction longitudinale de l'article et le long de la ligne médiane de l'article, en conséquence de quoi, l'article est, lors de l'utilisation, muni d'un pli dans la direction longitudinale de l'article le long dudit deuxième trou (620), lequel pli s'étend dans la rainure entre les fesses de la personne utilisatrice lors de l'utilisation de l'article, et maintient ainsi l'article en place de façon stable sur la personne utilisatrice.

12. Article selon la revendication 11, **caractérisé en ce que** ladite échancrure (13) a une forme de coin, est située symétriquement et forme un angle (β) compris entre 10° et 120°, de préférence entre 15° et 40°, à son extrémité qui fait face à la partie entrejambe.

13. Article selon l'une quelconque des revendications 8 à 12, **caractérisé en ce que** la deuxième zone partielle (62) est constituée du même matériau que la première zone partielle (61).

14. Article selon l'une quelconque des revendications 8 à 13, **caractérisé en ce que** le deuxième élément partiel (62) est réalisé avec la même rigidité que le premier élément partiel (61).

15. Article selon l'une quelconque des revendications qui précèdent, **caractérisé en ce que** le premier élément partiel de raidissement (61) a une rigidité à l'état sec de l'ordre de 1 à 15 N, mesurée selon la norme ASTMD 4032-82.

16. Article selon l'une quelconque des revendications qui précèdent, **caractérisé en ce que** lesdites zones partielles de raidissement (61, 62) consistent en des zones partielles comprimées d'un corps de matériau continu réalisé en une seule pièce, qui forme ladite zone d'entrejambe absorbante (63) entre lesdites zones partielles.

17. Article selon l'une quelconque des revendications qui précèdent, **caractérisé en ce que** lesdites zones partielles de raidissement (61, 62) sont constituées de zones partielles d'un corps réalisé en une seule pièce à partir d'un matériau du type mousse, lesquelles zones partielles sont comprimées et collées à l'état comprimé.

18. Article selon l'une quelconque des revendications 1 à 16, **caractérisé en ce qu'**au moins une desdites zones partielles de raidissement est constituée d'un élément partiel séparé.

19. Article selon la revendication 18, **caractérisé en ce que** ladite zone partielle de raidissement (61, 62) sert également d'élément absorbant et consiste en un certain nombre de couches de papier absorbant interconnectées, du matériau superabsorbant, sous forme de particules ou de fibres étant disposé entre les couches.

20. Article selon l'une quelconque des revendications 1 à 16, **caractérisé en ce que** la première zone partielle de raidissement (61) est constituée d'un mat de fibres formé à sec d'une masse volumique comprise entre 0,15 g/cm³ et 0,75 g/cm³ et une masse surfacique de l'ordre de 100 g/m² à 400 g/m².

21. Article selon la revendication 20, **caractérisé en ce que** le mat de fibres formé à sec est, après compression, mécaniquement assoupli jusqu'à atteindre la rigidité désirée.

22. Article selon les revendications 20 à 21, **caractérisé en ce que** le mat de fibres formé à sec est doté de la rigidité réduite désirée et de l'extensibilité désirée grâce au degré de compression choisi et au motif de compression choisi.

23. Article selon l'une quelconque des revendications qui précèdent, **caractérisé en ce que** la première zone partielle de raidissement (61) a une rigidité d'au moins 1,0 N, et **en ce que** la première zone partielle de raidissement est conçue de manière à avoir essentiellement la même rigidité sur toute l'étendue de la zone partielle de raidissement.

24. Article selon l'une quelconque des revendications qui précèdent, **caractérisé en ce que** les bords latéraux du premier élément partiel de raidissement (61) qui divergent depuis la partie entrejambe (3) sur au moins une partie de l'étendue de la partie avant (1) de l'article, sont agencés de manière à former un angle (α) entre une ligne s'étendant dans la direction longitudinale de l'article et chacun desdits bords latéraux, angle qui est de l'ordre de 35° à 55°, de préférence de l'ordre de 45°.

25. Article selon l'une quelconque des revendications 8 à 24, **caractérisé en ce que** l'élément d'entrejambe absorbant (63) est fixé à la deuxième zone partielle de raidissement (62) et est maintenue en place par cette dernière de manière à éviter que l'article glisse vers l'avant lorsque la personne utilisatrice se déplace.

26. Article selon l'une quelconque des revendications qui précèdent, **caractérisé en ce que** ladite fixation de la première zone partielle de raidissement à la zone d'entrejambe consiste en une connexion obtenue par l'intermédiaire d'un élément de connexion faisant partie de l'article.

27. Article selon la revendication 26, **caractérisé en ce que** ledit élément de connexion consiste en la couche (5) étanche aux liquides.

28. Article selon la revendication 26, **caractérisé en ce que** ledit élément de connexion est constitué d'un élément extensible élastiquement (64), en conséquence de quoi la zone d'entrejambe (63) peut se déplacer par rapport à la première zone partielle (61) et, grâce à l'action de l'élastique, avoir tendance à revenir à sa position d'origine.

29. Article selon l'une quelconque des revendications 25 à 28, **caractérisé en ce que** ladite fixation de la deuxième zone partielle de raidissement (62) à la zone d'entrejambe consiste en une connexion obtenue par l'intermédiaire d'un élément de connexion faisant partie de l'article.

30. Article selon la revendication 29, **caractérisé en ce que** ledit élément de connexion consiste en la couche (5) étanche aux liquides.

31. Article selon la revendication 28, **caractérisé en ce que** ledit élément de connexion est constitué d'un élément extensible élastiquement, en conséquence de quoi la partie entrejambe peut se déplacer par rapport à la deuxième zone partielle et, grâce à l'action de l'élastique, avoir tendance à revenir à la sa position d'origine.

32. Article selon l'une quelconque des revendications qui précèdent, **caractérisé en ce qu'**un élément formant une partie bombée (24), constitué d'un matériau élastique est disposé sous la zone d'entrejambe absorbante (63) sur au moins une partie de celle-ci, lequel élément formant une partie bombée (24) est agencé de manière à présenter une partie en relief (240) du côté où il doit être adapté au corps de la personne utilisatrice, la partie en relief étant agencée de manière à venir se placer directement en face des parties génitales de la personne utilisatrice.

33. Article selon la revendication 32, **caractérisé en ce que** la partie en relief (240) est oblongue dans la direction longitudinale de l'article et a une longueur comprise entre 20 mm et 120 mm.

34. Article selon la revendication 32 ou 33, **caractérisé en ce que** la partie en relief (240) est plus étroite que le reste de l'article dans la zone d'entrejambe et **en ce que** la partie en relief est plus épaisse que les zones environnantes.

35. Article selon l'une quelconque des revendications qui précèdent, **caractérisé en ce qu'**un moyen élastique (16) est agencé dans la direction longitudinale de l'article et de façon centrale le long de la partie arrière (2) de l'article et le long d'au moins une partie de celle-ci, depuis la partie entrejambe (3), lequel moyen élastique est destiné, sur sa longueur, à rassembler des parties de matériau adjacentes et à courber l'article vers le haut en vue d'obtenir un meilleur contact avec le corps de la personne utilisatrice.

36. Article selon l'une quelconque des revendications qui précèdent, **caractérisé en ce que** la première zone partielle de raidissement (61) sert également d'élément absorbant et est essentiellement homogène sur toute son étendue en ce qui concerne l'épaisseur, la rigidité, la capacité d'étalement et la capacité d'absorption, en conséquence de quoi la première zone partielle se courbe de façon uniforme lors de l'utilisation de l'article sans former des irrégularités locales qui pourraient donner lieu à des dispersions indésirables de liquide.

37. Article selon l'une quelconque des revendications qui précèdent, **caractérisé en ce que** la deuxième zone partielle de raidissement (62) sert également d'élément absorbant et est essentiellement homogène sur toute son étendue en ce qui concerne l'épaisseur, la rigidité, la capacité d'étalement et la capacité d'absorption, en conséquence de quoi la deuxième zone partielle se courbe de façon uniforme lors de l'utilisation de l'article sans former des irrégularités locales qui pourraient donner lieu à des dispersions indésirables de liquide.

38. Article selon l'une quelconque des revendications qui précèdent, **caractérisé en ce que** la largeur de la zone d'entrejambe (63) en arrière de ladite zone de transition (12) augmente de façon continue dans la partie entrejambe (3) en direction de l'arrière, vers la partie arrière (2) dans le but d'utiliser de façon optimale l'espace de largeur disponible dans cette zone en ce qui concerne l'absorption maximum.

39. Article selon l'une quelconque des revendications qui précèdent, **caractérisé en ce que** l'article est agencé, grâce à la rigidité choisie pour la première zone partielle de raidissement (61) et grâce au choix de la géométrie et des dimensions dans la transition (12) et autour de celle-ci, entre la partie entrejambe (3) et la partie avant (1), lorsque l'article est mis en place en relation avec le fait qu'il est porté, la transition entre la partie avant et la partie entrejambe étant placée entre lesdits tendons musculaires, de manière à ce qu'il soit fixé entre ceux-ci et ainsi, subisse une transformation à partir de la forme plane en une forme tridimensionnelle, la partie avant étant courbée vers le haut par rapport à la partie entrejambe et adoptant une forme de coupe au moins dans une zone proche de la partie entrejambe.

40. Article selon l'une quelconque des revendications qui précèdent, **caractérisé en ce que** la première zone partielle de raidissement (61) comporte un premier trou traversant (610) qui est oblong et qui s'étend dans la direction longitudinale de l'article et le long de la ligne médiane de l'article et qui rend possible une compression élastique dans la direction latérale de la première zone partielle lorsque des forces latérales agissent contre les bords latéraux de la première zone partielle.

41. Article selon l'une quelconque des revendications qui précèdent, **caractérisé en ce que** la zone d'entrejambe absorbante (63) a une longueur (G) de l'ordre de 70 mm à 120 mm.

42. Article selon la revendication 10, **caractérisé en ce que** l'article consiste en une couche, **en ce qu'**un élastique qui est transversal par rapport à la direction longitudinale de l'article, est agencé dans la partie arrière en travers de ladite échancrure (13) et **en ce que** l'élastique transversal est agencé de manière à amener le matériau de couverture de l'article dans ladite échancrure, en conséquence de quoi une poche extensible destinée à recevoir des matières fécales est formée dans ladite échancrure (13).
